# EUROPÄISCHE PATENTANMELDUNG

(11) **EP 0 974 639 A2**
(43) Veröffentlichungstag der Anmeldung: **26.01.2000**
(21) Anmeldenummer: 99113481.8
(22) Anmeldetag: 13.07.1999
(51) Int. Cl.: C11C 1/00, A61K 7/00, A61K 35/64, A61K 7/027, C11D 10/04, A61K 7/06, A61K 7/075, A61K 7/08, A61K 7/11, A61K 7/15, A61P 17/10

(54) **Verfahren zur Isolierung und Reinigung von Fettsäuren und Hydroxyfettsäuren aus Insektenwachsen und deren Verwendung**

(30) Priorität: 22.07.1998 DE 19832889
(71) Anmelder: Beiersdorf Aktiengesellschaft, 20245 Hamburg (DE)
(72) Erfinder: Hoppe, Udo, Prof. Dr., 24598 Heidmühlen (DE); Wolf, Florian, Dr., 20251 Hamburg (DE); Jacob, Jürgen, Prof. Dr., 22397 Hamburg (DE)

(57) **Zusammenfassung**

Die Erfindung betrifft ein neues Verfahren zur Isolierung und Reinigung von Hydroxyfettsäuren und Fettsäuren ausgehend von Insektenwachssäuregemischen sowie Verwendungen von Hydroxyfettsäuren und/oder Fettsäuren und ihren Salzen und kosmetische, dermatologische oder medizinische Zubereitungen, die sie enthalten.

## Beschreibung

Die vorliegende Erfindung betrifft ein neues Verfahren zur Isolierung und Reinigung von Hydroxyfettsäuren und Fettsäuren ausgehend von Insektenwachsgemischen sowie die Verwendungen von aus Insektenwachsgemischen gewonnenen langkettigen Fettsäuren und/oder Hydroxyfettsäuren und ihren Salzen sowie kosmetischen und/oder dermatologischen Zubereitungen, dieselben enthaltend, als gegen Bakterien, Mykota, Viren, Parasiten und Protozoen gerichtete und/oder hautpflegende Wirkstoffe.

Wachse sind Fettsäureester, die in tierischen und pflanzlichen Produkten vorkommen, sich aber von den Fetten und Ölen dadurch unterscheiden, daß die zugrundeliegenden Fettsäuren nicht mit Glycerin, sondern mit höheren, primären, einwertigen Alkoholen verestert sind.

Bienenwachs z. B. ist ein Ausscheidungsprodukt aus Drüsen der Honigbienen, das diese zum Bauen der Honigwaben verwenden. Gelbes (Cera flava), braunes oder rotes sogenanntes Rohwachs ist beispielsweise erhältlich, indem man die vom Honig durch Ausschleudern befreiten Waben schmilzt, die Schmelze (Schmp. 60-82° C) von festen Verunreinigungen trennt und das so erhaltene Rohwachs erstarren läßt. Das Rohwachs kann durch Behandlung mit Oxidationsmitteln vollkommen weiß gebleicht werden (Cera alba).

Bienenwachs besteht aus dem in Alkohol leichtlöslichen Cerin, einem Gemisch aus Cerotinsäure CH₃(CH₂)₂₄COOH und Melissinsäure CH₃(CH₂)₂₈COOH sowie aus einem Myricin genannten Ester-Gemisch aus ca. 70 Estern von C₁₆- bis C₃₆-Säuren und C₂₄-bis C₃₆-Alkoholen. Als wesentliche Bestandteile von Bienenwachs finden sich Myricylpalmitat 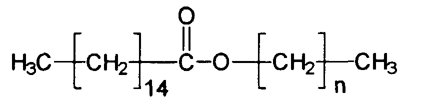 (mit 29 ≤n≤31), Myricylcerotinat 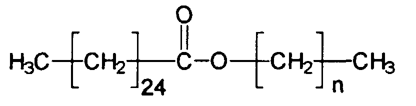 (mit 29 ≤n≤31) und Paraffin.

Auch andere Insektenwachse wie beispielsweise Hummelwachs oder Chinawachs sind im wesentlichen Mischungen verschiedener Ester. Chinawachs z. B. wird in China und Japan von der auf der chinesischen Esche lebenden Wachsschildlaus (Coccus ceriferus) und den Schildlausarten Ceroplastes ceriferus und Ericerus pela abgeschieden bzw. erzeugt. Es wird von den Bäumen abgekratzt und durch Umschmelzen in kochendem Wasser gereinigt. Hauptbestandteil des Chinawachses ist der Cerotinsäureester des Cerylalkohols:

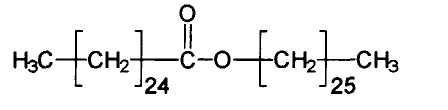

Insbesondere Bienenwachs, aber auch Chinawachs ist in kosmetischen oder dermatologischen Präparaten, Salben, Ceraten und Suppositorien zum Teil tragender Bestandteil. Bienenwachs ist ein schlechter Emulgator, weshalb Präparate auf Bienenwachs-Basis keine echten Emulsionen, sondern nur Pseudoemulsionen vom Typ W/O darstellen. Diese Emulsionen zerfallen auf der Haut und lassen das Wasser verdunsten, wodurch es zu einer Kühlwirkung kommt. Diesem Umstand ist es zu verdanken, daß eine Bienenwachs-haltige Salbe, das Unguentum leniens, auch heute noch als Kühlsalbe verwendet wird.

Von den Insektenwachsen ist insbesondere Bienenwachs, das in verschiedener Hinsicht gewisse nachteilige Eigenschaften aufweist, Gegenstand mehrfacher technologischer Bemühungen gewesen, deren Ergebnis eine Reihe von Derivaten und Aufbesserungsprodukten ist.

Zahlreiche Verfahren zur Anreicherung der Komponenten von Insektenwachsen sind in den letzten Jahrzehnten beschrieben worden. Hierbei handelt es sich häufig um destillative Verfahren, die in der Regel gering reproduzierbare Qualitäten liefern und darüber hinaus eine Trennung bzw. Isolation der Komponenten nur unbefriedigend gestatten. Die für Insektenwachse bekannten Verfahren haben den Nachteil, daß sie oft nur als Teilschritte, beispielsweise zur Vorreinigung geeignet sind. Nach keinem dieser bekannten Verfahren lassen sich große Mengen von Insektenwachsgemischen auf wirtschaftliche Weise in die Komponenten zerlegen.

Aufgabe der Erfindung ist es, ein Verfahren zur Isolierung und Reinigung von Hydroxyfettsäuren und Fettsäuren aus Insektenwachsgemischen zu schaffen, das die Nachteile des Standes der Technik nicht aufweist. Es soll in wenigen, einfachen Schritten auch von technischen Insektenwachsgemischen ausgehend, zu den genannten Komponenten führen und ein wirtschaftliches Aufarbeiten auch großer Mengen gestatten.

Dabei sollen die Produkte bzw. Gemische weitgehend rein, kristallin und geruchlos anfallen, so daß ihre direkte Weiterverarbeitung auch in empfindlichen Zubereitungen wie Kosmetika oder Dermatika möglich ist.

Diese Aufgabe wird gelöst durch ein Verfahren zur Isolierung und Reinigung von Hydroxyfettsäuren und Fettsäuren aus Insektenwachshydrolysaten, welche nach Hydrolyseverfahren aus Insektenwachsen bzw. Insektenwachsgemischen erhalten werden, das dadurch gekennzeichnet ist, daß man
a) in einem ersten Schritt ein Insektenwachshydrolysat mit mindestens einem polaren Lösungsmittel behandelt, wobei sich ein Teil des Gemisches löst, die erhaltene Lösung von ungelösten Anteilen abtrennt, wobei die ungelösten Anteile aus vorwiegend nicht hydroxylierten, langkettigen höheren Fettsäuren bestehen und durch physikalische und/oder chemische Trennverfahren abge-trennt werden können, und daß man dann das Lösungsmittel aus der verbleibenden Lösung entfernt und einen weiteren Rückstand erhält, der die Hydroxyfettsäuren enthält, und gegebenenfalls in einem zweiten Schritt diesen erhaltenen Rückstand mit mindestens einem unpolaren Lösungsmittel behandelt, wobei sich eine Dispersion oder Suspension bildet, deren festen Anteil, der die Hydroxyfettsäuren enthält, davon abtrennt und die verbleibende flüssige Phase vom Lösungsmittel befreit, wodurch man einen Rückstand erhält, der vorwiegend aus nicht hydroxylierten, kurzkettigen höheren Fettsäuren besteht, oder in umgekehrter Reihenfolge
b) in einem ersten Schritt ein Insektenwachshydrolysat mit mindestens einem unpolaren Lösungsmittel behandelt, wobei sich eine Dispersion oder Suspension bildet, den festen Anteil, der die Hydroxyfettsäuren enthält, davon abtrennt und die verbleibende flüssige Phase vom Lösungsmittel befreit, wobei ein Rückstand entsteht, der vorwiegend aus nicht hydroxylierten, langkettigen höheren Fettsäuren besteht, und gegebenenfalls in einem zweiten Schritt den verbliebenen festen Anteil mit den Hydroxyfettsäuren mit mindestens einem polaren Lösungsmittel behandelt, wobei sich ein Teil des festen Anteiles löst, die erhaltene Lösung von ungelösten Teilen, die vorwiegend aus nicht hydroxylierten höheren Fettsäuren kurzer Kettenlänge bestehen, befreit und von der Lösung das Lösungsmittel entfernt, wodurch man einen Rückstand erhält, der die Hydroxyfettsäuren enthält, und
c) gegebenenfalls zur Reinigung jeweils die zuvor erhaltenen Hydroxyfettsäuren oder Fettsäuren in wäßriger Lösung mit einer Base in eine Lösung ihrer Salze überführt, die Lösung von festen Anteilen befreit, dann nach Säurezugabe zur Lösung die Hydroxyfettsäure oder Fettsäure wiedergewinnt und isoliert, oder diese Reinigung gegebenenfalls auch am Anfang, vor den Schritten a) oder b), in entsprechender Weise mit dem Insektenwachshydrolysat vornimmt.

Bevorzugt wird die unter a) beschriebene Reihenfolge der Einzelschritte. Das Insektenwachshydrolysat wird also zuerst mit dem polaren Lösungsmittel und dann mit dem unpolaren Lösungsmittel wie angegeben behandelt.

Die in den Verfahrensteilen a) und b) - also die jeweils im ersten Schritt - isolierten Gemische enthalten Fettsäuren und/oder (ω-1)-Hydroxyfettsäuren in höheren Gewichtsanteilen als in den Ausgangsgemischen und können z.B. in kosmetischen, dermatologischen oder medizinischen Zubereitungen verwendet werden. Vorzugsweise schließt sich an die Trennungs- und Isolierungsschritte des Verfahrens aber der fakultative Reinigungsschritt an, der zu einer weiteren Anreicherung der Fettsäuren und/oder (ω-1)-Hydroxyfettsäuren führt.

Dies kann dann besonders zweckmäßig sein, wenn die erhaltenen Hydroxyfettsäuren und Fettsäuren in kosmetischen, dermatologischen oder medizinischen Zubereitungen verwendet werden. Auf diese Weise können noch aus dem Insektenwachs oder der Insektenwachsspaltung herrührende Verunreinigungen entfernt werden. Es ist aber auch möglich, diesen Reinigungsschritt vor Beginn der Isolierungsschritte a) oder b) vorzunehmen.

Das Verfahren bezieht sich vorzugsweise auf die Isolierung und Reinigung von (ω-1)-Hydroxyfettsäuren (nachfolgend auch (ω-1)-Hydroxysäuren genannt) und langkettigen, unverzweigten Fettsäuren mit Kettenlängen von 18 bis 26 Kohlenstoffatomen.

(ω-1)-Hydroxyfettsäuren im Sinne der vorliegenden Erfindung zeichnen sich durch die folgende chemische Struktur aus:

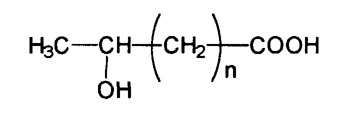

Die als Ausgangsprodukt verwendeten bekannten Insektenwachse bzw. Insektenwachsgemische sind als natürliche Produkte unterschiedlich zusammengesetzt. Besonders bevorzugt können Insektenwachsgemische aus Bienenwachsen unterschiedlicher Herkunft verwendet werden.

Zur Verseifung oder Hydrolyse werden die Insektenwachse bzw. Insektenwachsgemische in roher und/oder vorgereinigter - beispielsweise gebleichter - Form in an sich bekannter Weise, beispielsweise mit einer Base, vorzugsweise Alkalihydroxiden wie beispielsweise Natriumhydroxid (NaOH) und/oder Kaliumhydroxid (KOH) in Alkoholen, Wasser oder Gemischen davon, vorzugsweise aber in Alkoholen wie Methanol oder Ethanol, insbesondere bei pH 12 bis 14, verseift. Besonders bevorzugt ist die Verwendung von alkoholischer KOH-Lösung, insbesondere von ethanolischer Kalilauge.

Vorteilhafte Insektenwachshydrolysate im Sinne der vorliegenden Erfindung werden ebenfalls durch die saure Hydrolyse von Insektenwachsen bzw. Insektenwachsgemischen erhalten, beispielsweise mit anorganischen Mineralsäuren, wie z.B. Borsäure, Salzsäure, salpetrige und/oder Salpetersäure, schweflige und/oder Schwefelsäure, den verschiedenen Phosphorsäuren und dergleichen mehr und/oder mit sauren Salzen der Elemente der 1. bis 3. Hauptgruppe und/oder mit organischen Säuren, wie z.B. Ameisensäure und/oder Essigsäure.

Durch die Spaltung erhält man im wesentlichen zwei Fraktionen, von denen die eine vorwiegend Alkohole und die andere vorwiegend Fettsäuren und Hydroxyfettsäuren enthält.

Eine Trennung der beiden Fraktionen voneinander erfolgt beispielsweise, indem zu der durch die alkalische Spaltung erhaltenen Mischung Wasser und ein mit Wasser nicht mischbares Lösungsmittel, wie beispielweise halogenfreie, z.B. Petrolether oder n-Heptan, oder halogenierte, insbesondere chlorierte Kohlenwasserstoffe, hinzugegeben und die unverseifbaren Anteile ausgeschüttelt werden. Ein bevorzugt zu verwendendes, mit Wasser nicht mischbares Lösungsmittel ist Trichlormethan.

Anschließend wird die wäßrige Phase mit einer Säure, vorzugsweise mit einer Mineralsäure, insbesondere 1/10 bis 1/2-normaler Salzsäure, angesäuert und vorzugsweise so viel Wasser bzw. Säure zugegeben, bis ein pH-Wert von etwa 3 vorliegt.

Die sich abscheidende Säure wird abfiltriert oder vorzugsweise mit nicht wassermischbaren Lösungsmitteln wie halogenfreien, z.B. Petrolether oder n-Heptan, oder halogenierten, insbesondere chlorierten Kohlenwasserstoffen, z.B. Trichlormethan extrahiert.

Auf die beschriebene, erfindungsgemäße Weise erhält man etwa 5 bis 65 Gewichtsprozent bezogen auf das Ausgangsgewicht eines gereinigten Säuregemisches, insbesondere Hydroxyfettsäuregemische und/oder Fettsäuregemisches.

Das erfindungsgemäße Verfahren liefert vorzugsweise Insektenwachssäurefraktionen, die zwischen 10 und 90 Gew.-% (ω-1)-Hydroxysäuren enthalten, die eine Kettenlänge von C₁₀ bis C₂₀ haben. (ω-2)-Hydroxysäuren finden sich dagegen abhängig vom Ausgangsprodukt nur in geringem Maß. Es treten sowohl geradkettige als auch verzweigte (ω-1)-Hydroxysäuren sowie normale und iso-(ω-1)-Hydroxysäuren in wechselnden Anteilen auf, wobei das Verteilungsmaximum der (ω-1)-Hydroxysäuren bei der (ω-1)-Hydroxyhexadecansäure liegt. Die aus dem natürlichen Insektenwachs stammenden (ω-1)-Hydroxysäuren werden in optisch aktiver Form erhalten. Sie liegen vorzugsweise als D-Enantiomere vor.

Die nach dem erfindungsgemäßen Verfahren erhaltenen Insektenwachssäurefraktionen enthalten gleichzeitig zwischen 90 und 10 Gew.-% langkettige, unverzweigte Fettsäuren mit Kettenlängen von 18 bis 26 Kohlenstoffatomen. Es handelt sich vorzugsweise um Fettsäuren mit einer Kohlenstoffzahl von 20 bis 24, wobei das Verteilungsmaximum bei C₂₄ liegt.

Gegenstand der Erfindung sind auch die nach dem erfindungsgemäßen Verfahren erhaltenen oder erhältlichen Insektenwachsfraktionen, insbesondere solche, die einen hohen Gehalt an (ω-1)-Hydroxysäuren und/oder unverzweigten Fettsäuren mit einer Kettenlänge von C₁₈ bis C₂₆ aufweisen.

Die verwendeten Lösungsmittel werden vorzugsweise rein oder technisch rein eingesetzt und sind weitgehend wasserfrei. Es können einzelne Lösungsmittel oder auch Gemische, vorzugsweise von 2 bis 5 Lösungsmitteln, insbesondere 2 oder 3 Lösungsmitteln verwendet werden.

Die Ausgangsprodukte und überraschenderweise auch die in den Zwischenstufen des Verfahrens erhaltenen Säuregemische liegen zumeist wachsartig bis kristallin vor, und sie lassen sich unter üblichen Verfahrensbedingungen und Einsatz üblicher Mechanik wirkungsvoll bearbeiten, insbesondere bei Raumtemperatur.

Das erfindungsgemäße Verfahren liefert überraschend mit wenigen einfachen Schritten weitgehend reine Komponenten der Insektenwachsgemische und dies auf wirtschaftliche Weise. Sie können z.B. direkt in Kosmetika weiterverarbeitet werden oder als Ausgangsverbindungen zur Herstellung wertvoller Derivate dienen.

Alle Prozentangaben oder Teile beziehen sich immer, soweit nicht anders angegeben, auf das Gewicht, insbesondere auf das Gesamtgewicht der Zubereitungen.

### Verwendungen von Hydroxyfettsäuren und/oder Fettsäuregemischen als gegen Bakterien, Mykota, Viren, Parasiten und Protozoen gerichtete sowie zur Hautpflege geeignete Wirkstoffe sowie Zubereitungen, dieselben enthaltend

Gegenstand der Erfindung sind auch die Verwendung von Fettsäuren und/oder (ω-1)-Hydroxyfettsäuren und/oder Insektenwachsgemischen und/oder Insektenwachshydrolysaten und/oder Insektenwachsfraktionen aus Insektenwachsen, insbesondere solchen, die einen hohen Gehalt an Fettsäuren und/oder (ω-1)-Hydroxysäuren aufweisen, als gegen Bakterien, Mykota, Viren, Parasiten und Protozoen gerichtete Wirkstoffe sowie stabile kosmetische, dermatologische oder medizinische Mittel, insbesondere Mittel mit hautpflegenden und/oder barrierestärkenden Eigenschaften, die diese enthalten. Die Anwendung der Wirkstoffe kann topisch, perkutan, transdermal, parenteral, oral oder auch intravasal erfolgen.

Zubereitungen, die erfindungsgemäße Wirkstoffe enthalten, können topische Zubereitungen sein, beispielsweise kosmetische und dermatologische topische Zubereitungen oder aber auch übliche Arzneimittel-Darreichungsformen. Bevorzugt werden Desodorantien oder desodorierende Körperreinigungsprodukte oder Körperpflegeprodukte. Die Wirkstoffe können aber auch in Desinfektionsmitteln und/oder Reinigungsmitteln enthalten sein, die nicht nur zur Behandlung des Körpers oder der Haut bestimmt sind, sondern auch zum Reinigen und Desinfizieren von harten Oberflächen, medizinischen Materialien, Geräten, Instrumenten, Mobiliar und Wänden.

Für den Körper bestimmte Reinigungsmittel, Desinfektionsmittel, Spülmittel können ebenfalls zur Behandlung von der Haut verwendet werden wie schon die topischen Zubereitungen. Sie dienen aber vorzugsweise zur Behandlung von Körperhöhlen, Wunden und auch des Mund- und Rachenraumes sowie der Nase.

Die Wirkstoffe gemäß der Erfindung können mit üblichen pharmazeutisch verträglichen Verdünnungsmitteln oder Trägern und gegebenenfalls mit anderen Hilfsmitteln vermischt und beispielsweise oral oder parenteral verabreicht werden. Sie können vorzugsweise oral in Form von Granulaten, Kapseln, Pillen, Tabletten, Filmtabletten, Dragees, Sirupen, Emulsionen, Suspensionen, Dispersionen, Aerosolen und Lösungen sowie Flüssigkeiten oder auch als Zäpfen, Vaginalkugeln oder parenteral z.B. in Form von Lösungen, Emulsionen oder Suspensionen verabreicht werden. Oral zu verabreichende Präparate können einen oder mehrere Zusätze wie Süßungsmittel, Aromatisierungsmittel, Farbstoffe und Konservierungsmittel enthalten. Tabletten können den Wirkstoff mit üblichen pharmazeutisch verträglichen Hilfsmitteln vermischt enthalten, zum Beispiel inerten Verdünnungsmitteln wie Calciumcarbonat, Natriumcarbonat, Lactose und Talk, Granulierungsmitteln und Mitteln, die den Zerfall der Tabletten bei oraler Verabreichung fördern wie Stärke oder Alginsäure, Bindemitteln wie Stärke oder Gelatine, Gleitmitteln wie Magnesiumstearat, Stearinsäure und Talkum.

Geeignete Trägerstoffe sind beispielsweise Milchzucker (Lactose), Gelatine, Maisstärke, Stearinsäure, Ethanol, Prophylenglycol, Ether des Tetrahydrofurfurylalkohols und Wasser.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calziumcarbonat und Dicalziumphosphat zusammen mit verschiedenen Zuschlagstoffen, wie Stärke, vorzugsweise Kartoffelstärke, Gelatine und dergleichen enthalten. Weiterhin können Gleitmittel wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer mit den obengenannten Hilfsstoffen mit verschiedenen Geschmacksaufbesserem oder Farbstoffen versetzt werden.

Für den Fall der parenteralen Anwendung können Lösungen der Wirkstoffe unter Verwendung geeigneter flüssiger Trägermaterialien eingesetzt werden.

Kapseln können den Wirkstoff als einzigen Bestandteil oder vermischt mit einem festen Verdünnungsmittel wie Calciumcarbonat, Calciumphosphat oder Kaolin enthalten. Die injizierbaren Präparate werden ebenfalls in an sich bekannter Weise formuliert.

Die pharmazeutischen Präparate können den Wirkstoff in einer Menge von 0,1 bis 90 Gewichtsprozent, insbesondere 1 - 90 Gew.-% enthalten. Kapseln werden besonders bevorzugt. Einzeldosen enthalten die Wirkstoffe vorzugsweise in einer Menge von 0,1 bis 10 g.

Die erfindungsgemäßen kosmetischen und dermatologischen Formulierungen können wie üblich zusammengesetzt sein und zur Behandlung der Haut und/oder der Haare im Sinne einer dermatologischen Behandlung oder einer Behandlung im Sinne der pflegenden Kosmetik dienen. Sie können aber auch in Schminkprodukten in der dekorativen Kosmetik eingesetzt werden oder in kosmetischen und dermatologischen Reinigungsprodukten.

Zur Anwendung werden die erfindungsgemäßen kosmetischen und dermatologischen Formulierungen in der für Kosmetika und Dermatika üblichen Weise auf die Haut und/oder die Haare in ausreichender Menge aufgebracht.

Unter Hautpflege im Sinne der Erfindung ist in erster Linie zu verstehen, daß die natürliche Funktion der Haut als Barriere gegen Umwelteinflüsse (z.B. Schmutz, Chemikalien, Mikroorganismen) und gegen den Verlust von körpereigenen Stoffen (z.B. Wasser, Lipide, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Die menschliche Haut verliert durch körpereigene Mechanismen wie die Transpiration, aber auch durch äußere Einflüsse wie die tägliche Körperwäsche, Wind und Wetter ständig eine gewisse Menge an Feuchtigkeit. Obwohl die gesunde Haut durchaus imstande ist, diesen Verlust auszugleichen, ist es Ziel der Hautpflege, die Haut beim Ausgleich dieses Verlustes zu unterstützen. Gerade dann aber, wenn das natürliche Regenerationsvermögen, zum Beispiel infolge einer Erkrankung, nicht ausreicht, ist die Regulation der Hautfeuchtigkeit unerläßlich.

Folgende Methoden der Hautpflege sind an sich bekannt:
a) Okklusion der Haut. Wird die Haut mit einem Lipidfilm (herkömmliche Salben oder Crèmes) bedeckt, so wird die Barrierefunktion der Haut nicht wiederhergestellt. Der Lipidfilm stellt nur eine physikalische Barriere dar, die verhindert, daß die Haut austrocknet. Allerdings kann dieser Film mit Reinigungsmitteln leicht abgewaschen werden, so daß wieder der ursprüngliche, beeinträchtigte Zustand erreicht ist.
b) Behandlung der Haut mit essentiellen Fettsäuren. Essentielle Fettsäuren werden derzeit gelegentlich in dermatologischen Präparaten zur Behandlung von trockener Haut eingesetzt. Eindeutige Beweise zu deren Wirksamkeit stehen aber noch aus.
c) Behandlung der Haut mit keratolytisch wirksamen Substanzen (z.B. Harnstoff, Salicylsäure, Hydroxycarbonsäuren usw.). Diese Substanzen wirken je nach verwendeter Konzentration keratolytisch, proteolytisch, wasserbindend, penetrationsfördernd, epidermisverdünnend oder juckreizstillend. Ihre Verwendung ist im wesentlichen auf medizinische Indikationen beschränkt.
d) Behandlung mit feuchtigkeitsregulierenden Substanzen (z.B. Glycerin, Sorbit, Allantoïn usw.) Die Gruppe dieser Substanzen ist von besonderem Interesse, da sie viele gut wirksame Vertreter enthält. Nachteilig ist indes, daß die meisten dieser Stoffe entweder kostspielig oder physiologisch bedenklich oder aber nur unter aufwendigen Bedingungen in kosmetische Zubereitungen einzuarbeiten sind. Zudem gilt für manche Vertreter dieser Gruppe das unter Punkt c) Gesagte.

Aufgabe der vorliegenden Erfindung war es also, die Nachteile des Standes der Technik zu beseitigen. Insbesondere sollten hautpflegende Zubereitungen zur Verfügung gestellt werden, welche die Barriereeigenschaften der Haut erhalten oder wieder herstellen und den durch die tägliche Reinigung der Haut verursachten Wasserverlust ausgleichen, zumal dann, wenn die natürliche Regeneration der Haut nicht ausreicht. Weiterhin soll die Haut vor Umwelteinflüssen wie Sonne und Wind geschützt werden. Aufgabe der vorliegenden Erfindung war es auch, stabile hautpflegende kosmetische und/oder dermatologische Mittel zur Verfügung zu stellen.

Medizinische topische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Die vorliegende Erfindung betrifft ferner Zubereitungen gegen Bakterien, Mycota, Viren, Parasiten und Protozoen. In besonderen Ausführungsformen betrifft die vorliegende Erfindung kosmetische und dermatologische Zubereitungen, solche Substanzen enthaltend.

Der gesunde warmblütige Organismus, insbesondere die gesunde menschliche Haut, ist mit einer Vielzahl nichtpathogener Mikroorganismen besiedelt. Diese sogenannte Mikroflora der Haut ist nicht nur unschädlich, sie stellt einen wichtigen Schutz zur Abwehr opportunistischer oder pathogener Keime dar.

Bakterien gehören zu den prokaryotischen Einzellern. Sie können grob nach ihrer Form (Kugel, Zylinder, gekrümmter Zylinder) sowie nach dem Aufbau ihrer Zellwand (grampositiv, gramnegativ) unterschieden werden. Feinere Unterteilungen tragen auch der Physiologie der Organismen Rechnung. So existieren aerobe, anaerobe sowie fakultativ anaerobe Bakterien. Manche Individuen sind in ihrer Eigenschaft als pathogene Keime von medizinischer Bedeutung, andere wiederum sind vollkommen harmlos.

Gegen Bakterien wirksame Substanzen sind seit geraumer Zeit bekannt. Allerdings sind antibakterielle Wirkstoffe, wie beispielsweise Antibiotika, nicht für alle medizinischen, schon gar nicht kosmetischen Anwendungen geeignet, da häufig auch der warmblütige Organismus, also etwa der erkrankte Patient, bei ihrer Anwendung auf irgendeine Weise in seinen Stoffwechselfunktionen beeinträchtigt wird.

Eine Aufgabe der vorliegenden Erfindung war also, den Stand der Technik in dieser Richtung zu bereichern, insbesondere also, Substanzen zur Verfügung zu stellen, welche gegen grampositive und/oder gramnegative Bakterien wirksam sind, ohne daß mit der Anwendung der Substanzen eine unvertretbare Beeinträchtigung der Gesundheit des Anwenders verbunden wäre.

Gramnegative Keime sind beispielsweise Escherichia coli, Pseudomonas-Arten sowie Enterobacteriaceen, wie etwa Citrobacter freundii.

Auch grampositive Keime spielen in Kosmetik und Dermatologie eine Rolle. Bei der unreinen Haut beispielsweise sind neben anderen Einflüssen bakterielle Sekundärinfektionen von ätiologischer Bedeutung. Einer der wichtigsten Mikroorganismen, der in Zusammenhang mit unreiner Haut steht, ist Propionibacterium acnes.

Unreine Haut und/oder Komedonen beeinträchtigen das Wohlbefinden der Betroffenen aber selbst in leichten Fällen. Da praktisch jeder oder jede Jugendliche von unreiner Haut irgendeiner Ausprägung betroffen ist, besteht großer Bedarf, diesem Zustande abzuhelfen.

Eine besondere Aufgabe der vorliegenden Erfindung war es also, einen gegen unreine Haut bzw. Propionibacterium acnes wirksamen Stoff bzw. Stoffkombination zu finden.

Die vorliegende Erfindung betrifft in einer weiteren Ausführungsform kosmetische Desodorantien. Solche Formulierungen dienen dazu, Körpergeruch zu beseitigen, der entsteht, wenn der an sich geruchlose frische Schweiß durch Mikroorganismen zersetzt wird. Den üblichen kosmetischen Desodorantien liegen unterschiedliche Wirkprinzipien zugrunde.

Schweiß erscheint an der Hautoberfläche als geruchloses, steriles Sekret. Erst durch die Verstoffwechselung bestimmter Schweißinhaltsstoffe durch die Bakterien der Achselflora entstehen Substanzen, die für den Achselgeruch verantwortlich sind. Folglich kann man durch den Einsatz von antimikrobiell wirksamen Substanzen in Deodorantien einen desodorierenden Effekt erzielen.

Bekannt und gebräuchlich sind sowohl flüssige Desodorantien, beispielsweise Aerosolsprays, Roll-ons und dergleichen als auch feste Zubereitungen, beispielsweise Deo-Stifte ("Sticks"), Puder, Pudersprays, Intimreinigungsmittel usw.

In sogenannten Antitranspirantien kann durch Adstringentien - vorwiegend Aluminiumsalze wie Aluminiumhydroxychlorid (Aluchlorhydrat) - die Entstehung des Schweißes unterbunden werden. Abgesehen von der Denaturierung der Hautproteine greifen die dafür verwendeten Stoffe aber, abhängig von ihrer Dosierung, drastisch in den Wärmehaushalt der Achselregion ein und sollten allenfalls in Ausnahmefällen angewandt werden.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. In der Praxis hat sich aber herausgestellt, daß die gesamte Mikroflora der Haut beeinträchtigt werden kann. Der Schweißfluß selbst wird dadurch nicht beeinflußt, im Idealfalle wird nur die mikrobielle Zersetzung des Schweißes zeitweilig gestoppt.

Auch die Kombination von Adstringentien mit antimikrobiell wirksamen Stoffen in ein und derselben Zusammensetzung ist gebräuchlich. Die Nachteile beider Wirkstoffklassen lassen sich auf diesem Wege jedoch nicht vollständig beseitigen.

Schließlich kann Körpergeruch auch durch Duftstoffe überdeckt werden, eine Methode, die am wenigsten den ästhetischen Bedürfnissen des Verbrauchers gerecht wird, da die Mischung aus Körpergeruch und Parfümduft eher unangenehm riecht.

Allerdings werden die meisten kosmetischen Desodorantien, wie auch die meisten Kosmetika insgesamt, parfümiert, selbst wenn sie desodorierende Wirkstoffe beinhalten. Parfümierung kann auch dazu dienen, die Verbraucherakzeptanz eines kosmetischen Produktes zu erhöhen oder einem Produkt ein bestimmtes Flair zu geben.

Die Parfümierung wirkstoffhaltiger kosmetischer Mittel, insbesondere kosmetischer Desodorantien, ist allerdings nicht sehen problematisch, weil Wirkstoffe und Parfümbestandteile gelegentlich miteinander reagieren und einander unwirksam machen können.

Desodorantien sollen folgende Bedingungen erfüllen:
1) Sie sollen eine zuverlässige Desodorierung bewirken.
2) Die natürlichen biologischen Vorgänge der Haut dürfen nicht durch die Desodorantien beeinträchtigt werden.
3) Die Desodorantien müssen bei Überdosierung oder sonstiger nicht bestimmungsgemäßer Anwendung unschädlich sein.
4) Sie sollen sich nach wiederholter Anwendung nicht auf der Haut anreichem.
5) Sie sollen sich gut in übliche kosmetische Formulierungen einarbeiten lassen.

Eine weitere Aufgabe der vorliegenden Erfindung war es also, kosmetische Desodorantien zu entwickeln, die die Nachteile des Standes der Technik nicht aufweisen. Insbesondere sollten die Desodorantien die Mikroflora der Haut weitgehend schonen, die Zahl der Mikroorganismen aber, die für den Körpergeruch verantwortlich sind, selektiv reduzieren.

Weiterhin war es eine Aufgabe der Erfindung, kosmetische Desodorantien zu entwickeln, die sich durch gute Hautverträglichkeit auszeichnen. Auf keinen Fall sollten die desodorierenden Wirkprinzipien sich auf der Haut anreichern.

Eine weitere Aufgabe war, kosmetische Desodorantien zu entwickeln, welche mit einer möglichst großen Vielzahl an üblichen kosmetischen Hilfs- und Zusatzstoffen harmonieren, insbesondere mit den gerade in desodorierend oder antitranspirierend wirkenden Formulierungen bedeutenden Parfümbestandteilen.

Noch eine weitere Aufgabe der Erfindung war, kosmetische Desodorantien zur Verfügung zu stellen, welche über einen längeren Zeitraum, und zwar in der Größenordnung von mindestens einem halben Tag, wirksam sind, ohne daß ihre Wirkung spürbar nachläßt.

Schließlich war eine Aufgabe der vorliegenden Erfindung, desodorierende kosmetische Prinzipien zu entwickeln, die möglichst universell in die verschiedensten Darreichungsformen kosmetischer Desodorantien eingearbeitet werden können, ohne auf eine oder wenige spezielle Darreichungsformen festgelegt zu sein.

Pilze, auch Fungi, Mycota oder Mycobionten genannt, zählen im Gegensatze zu den Bakterien zu den Eucaryonten. Eucaryonten sind Lebewesen, deren Zellen (Eucyten) im Gegensatz zu denen der sogenannten Procaryonten (Procyten) über einen durch Kernhülle und Kemmembran vom restlichen Cytoplasma abgegrenzten Zellkern verfügen. Der Zellkern enthält die Erbinformation in Chromosomen gespeichert.

Zu Vertretem der Mycobionten zählen beispielsweise Hefen (Protoascomycetes), Schimmelpilze (Plectomycetes), Mehltau (Pyrenomycetes), der falsche Mehltau (Phycomycetes) und die Ständerpilze (Basidiomycetes).

Pilze, auch nicht die Basidiomyceten, sind keine pflanzlichen Organismen, haben aber wie diese eine Zellwand, zellsaftgefüllte Vakuolen und eine mikroskopisch gut sichtbare Plasmaströmung. Sie enthalten keine photosynthetischen Pigmente und sind C-heterotroph. Sie wachsen unter aeroben Bedingungen und gewinnen Energie durch Oxidation organischer Substanzen. Einige Vertreter, beispielsweise Hefen, sind allerdings fakultative Anaerobier und zur Energiegewinnung durch Gärungsprozesse befähigt.

Dermatomycosen sind Krankheiten, bei der gewisse Pilzarten, insbesondere Dermatophyten, in die Haut und Haarfollikel eindringen. Die Symptome von Dermatomycosen sind beispielsweise Bläschen, Exfoliation, Rhagaden und Erosion, meist verbunden mit Juckreiz oder allergischem Ekzem.

Dermatomycosen können im wesentlichen in folgende vier Gruppen unterteilt werden: Dermatophytien (z.B. Epidermophytie, Favus, Mikrosporie, Trichophytie), Hefemycosen (z.B. Pityriasis und andere Pityrosporum-bedingte Mycosen, Candida-Infektionen, Blastomycose, Busse-Buschke-Krankheit, Torulose, Piedra alba, Torulopsidose, Trichosporose), Schimmelmycosen (z.B. Aspergillose, Kephalosporidose, Phycomycose und Skopulariopsidose), Systemmycosen (z.B. Chromomycose, Coccidiomycose, Histoplasmose).

Zu den pathogenen und fakultativ pathogenen Keimen gehören beispielsweise aus der Gruppe der Hefen Candida-Arten (z.B. Candida albicans) und solche der Familie Pityrosporum. Pityrosporum-Arten, insbesondere Pityrosporum ovale, sind für Hauterkrankungen wie Pityriasis versicolor. Seborrhoe in den Formen Seborrhoea oleosa und Seborrhoea sicca, welche sich vor allem als Seborrhoea capitis (= Kopfschuppen) äußern, seborrhoisches Ekzem und Pityrosporum-Follikulitis verantwortlich zu machen. Eine Beteiligung von Pityrosporum ovale an der Entstehung von Psoriasis wird von der Fachwelt diskutiert.

Alle Bereiche der menschlichen Haut können von Dermatomycosen befallen werden. Dermatophytien befallen fast ausschließlich Haut, Haare und Nägel. Hefemycosen können auch Schleimhäute und innere Organe befallen, Systemmycosen erstrecken sich regelmäßig auf ganze Organsysteme.

Besonders häufig sind die Körperbereiche betroffen, auf welchen sich durch Kleidung, Schmuck oder Schuhwerk Feuchtigkeit und Wärme stauen können. So gehört der Fußpilz zu den bekanntesten und am weitesten verbreiteten Dermatomycosen. Besonders unangenehm sind weiterhin Pilzerkrankungen der Finger- und Fußnägelbereiche (Onychomycosen).

Ferner sind Superinfektionen der Haut durch Pilze und Bakterien nicht selten.

Bei bestehendem Primärinfekt, d.h. der normalen Keimbesiedelung der Haut, eintretende Neuinfektion mit hohen Keimzahlen eines oder mehrerer oft physiologischer Erreger, beispielsweise Staphylokokken, oft aber auch unphysiologischer Erreger, beispielsweise Candida albicans, kann bei Zusammentreffen ungünstiger Einflüssen eine "Superinfektion" der befallenen Haut auftreten. Die normale Mikroflora der Haut (oder eines anderen Körperorgans) wird dabei von dem Sekundärerreger regelrecht überwuchert.

Solche Superinfektionen können sich, in Abhängigkeit vom betreffenden Keim, in günstig verlaufenden Fällen in unangenehmen Hauterscheinungen (Juckreiz, unschönes äußeres Erscheinungsbild) äußem. In ungünstig verlaufenden Fällen können sie aber zu großflächiger Zerstörung der Haut führen, im schlimmsten Falle sogar im Tode des Patienten gipfeln.

Superinfektionen der vorab geschilderten Art sind z.B. beim Vollbild von AIDS häufig auftretende Sekundärerkrankungen. An sich - jedenfalls in geringen Keimdichten - unschädliche, aber unter Umständen auch ausgesprochen pathogene Keime überwuchern auf diese Weise die gesunde Hautflora. Bei AIDS allerdings sind auch andere Körperorgane von Superinfektionen betroffen.

Ebenso werden derartige Superinfektionen bei einer Vielzahl dermatologischer Erkrankungen, z.B. atopischem Ekzem, Neurodermitis, Akne, seborrhoischer Dermatitis oder Psoriasis beobachtet. Auch viele medizinische und therapeutische Maßnahmen, z.B die Radio- oder Chemotherapie von Tumorerkrankungen, als Nebenwirkung hervorgerufene, medikamentös induzierte Immunsuppression oder aber systemische Antibiotikabehandlung, ebenso wie externe chemische oder physikalische Einflüsse (z.B. Umweltverschmutzung, Smog), fördem das Auftreten von Superinfektionen der äußeren und inneren Organe, insbesondere der Haut und der Schleimhäute.

Zwar ist es im Einzelfalle ohne weiteres möglich, Superinfektionen mit Antibiotika zu bekämpfen, meistens haben solche Substanzen aber den Nachteil unangenehmer Nebenwirkungen. Oft sind Patienten beispielsweise gegen Penicilline allergisch, weswegen eine entsprechende Behandlung sich in einem solchen Falle verbieten würde.

Ferner haben topisch verabreichte Antibiotika den Nachteil, daß sie die Hautflora nicht nur vom Sekundärerreger befreien, sondern auch die an sich physiologische Hautflora stark beeinträchtigen und der natürliche Heilungsprozeß auf diese Weise wieder gebremst wird.

Aufgabe der vorliegenden Erfindung war, die Nachteile des Standes der Technik zu beseitigen und Substanzen und Zubereitungen, solche Substanzen enthaltend, zur Verfügung zu stellen, durch deren Verwendung Superinfektionen geheilt werden können, wobei die physiologische Hautflora keine nenneswerte Einbußen erleidet.

Im Gegensatz zu den prokaryotischen und eukaryotischen zellulären Organismen sind Viren biologische Strukturen, welche zur Biosynthese eine Wirtszelle benötigen. Extrazelluläre Viren (auch 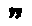 Virionen" genannt) bestehen aus einer ein- oder doppelsträngigen Nukleinsäuresequenz (DNS oder RNS) und einem Proteinmantel (dapsid genannt), gegebenenfalls einer zusätzlichen lipidhaltigen Hülle (Envelope) umgeben. Die Gesamtheit aus Nukleinsäure und Capsid wird auch Nucleocapsid genannt. Die Klassifikation der Viren erfolgte klassisch nach klinischen Kriterien, heutzutage allerdings zumeist nach ihrer Struktur, ihrer Morphologie, insbesondere aber nach der Nukleinsäuresequenz.

Medizinisch wichtige Virengattungen sind beispielsweise Influenzaviren (Familie der Orthomyxoviridae), Lyssaviren (z.B. Tollwut, Familie der Rhabdoviren) Enteroviren (z.B. Hepatitis-A, Familie der Picornaviridae), Hepadnaviren (z.B. Hepatitis-B, Familie der Hepadnaviridae).

Viruzide, also Viren abtötende Substanzen im eigentlichen Sinne gibt es nicht, da Viren nicht über eigenen Stoffwechsel verfügen. Es wurde aus diesem Grunde auch diskutiert, ob Viren als Lebewesen eingeordnet werden sollten. Pharmakologische Eingriffe ohne Schädigung der nicht befallenen Zellen sind jedenfalls schwierig. Mögliche Wirkmechanismen im Kampfe gegen die Viren sind in erster Linie die Störung ihrer Replikation, z.B. durch Blockieren der für die Replikation wichtigen Enzyme, die in der Wirtszelle vorliegen. Ferner kann das Freisetzen der viralen Nukleinsäuren in die Wirtszelle verhindert werden. Im Rahmen der hiermit vorgelegten Offenbarung wird unter Begriffen wie 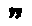 antiviral" oder 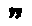 gegen Viren wirksam", 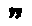 viruzid" oder ähnlichen die Eigenschaft einer Substanz verstanden, einen ein- oder mehrzeiligen Organismus vor schädlichen Folgen einer Virusinfektion, sei es prophylaktisch oder therapeutisch, zu schützen, ungeachtet dessen, was der tatsächliche Wirkmechanismus der Substanz im Einzelfalle sei.

Dem Stande der Technik mangelt es jedoch an gegen Viren wirksamen Substanzen, welche zudem den Wirtsorganismus nicht oder nicht in vertretbarem Maße schädigen.

Eine Aufgabe der vorliegenden Erfindung war also, diesem Übelstande abzuhelfen, also Substanzen zu finden, welche wirksam einen ein- oder mehrzelligen Organismus vor schädlichen Folgen einer Virusinfektion, sei es prophylaktisch oder therapeutisch, zu schützen.

Es ist bekannt, daß beispielsweise alpha-Hydroxyfettsäuren eine antimikrobielle Wirksamkeit gegenüber Bakterien, Mykota, Viren, Parasiten und Protozoen besitzen. Die deutsche Offenlegungsschrift DE 42 04 321 beschreibt in diesem Zusammenhang beispielsweise desodorierende kosmetische Mittel mit einem Gehalt an alpha-Hydroxyfettsäuren ausgehend von Wollwachssäuregemischen. Ganz erhebliche Nachteile von Wollwachssäuren sind allerdings deren starker Geruch und ihre Farbe. Der Einsatz von Wollwachssäuren, insbesondere in Kosmetika und Dermatika, erfordert daher große Formulierungssorgfalt, um Eigengeruch und -farbe zu überdecken. Darüber hinaus weisen Wollwachssäurefraktionen oder darin enthaltene einzelne Komponenten keine hautpflegenden und/oder die epidermale Barriere der Haut restaurierenden Eigenschaften auf.

In der Literatur existieren auch Hinweise bezüglich der antimikrobiellen Wirksamkeit von Bienenwachs (*S. L. Puleo, Cosmet Toiletries (1991), 106 (2), 83-89).*

So wurden verschiedene Bakterienspezies auf ihre Sensitivität gegenüber Bienenwachsextrakten getestet. Die antimikrobielle Aktivität des Bienenwachses wurde dabei auf Komponenten zurückgeführt, die im Wachs in Konzentrationen von weniger als 1 Gew.-% vorliegen, namentlich auf ein bestimmtes Flavonoid, so daß diese Untersuchungen nicht auf die vorliegende Erfindung hinweisen konnten. Insektenwachse in roher und/oder vorgereinigter - beispielsweise gebleichter - Form zeigen keine antimikrobielle Wirksamkeit.

Überraschenderweise werden die Aufgaben der vorliegenden Erfindung gelöst durch die Verwendung von (ω-1)-Hydroxyfettsäuren, insbesondere (ω-1)-Hydroxysäuren mit 10 bis 20 Kohlenstoffatomen, und/oder deren Salzen als gegen Bakterien, Mykota, Viren, Parasiten und Protozoen gerichtete Wirkstoffe, wobei die (ω-1)-Hydroxysäuren sowohl einzeln als auch im Gemisch vorliegen können, sowie durch kosmetische, dermatologische oder medizinische Zubereitungen, insbesondere Zubereitungen mit hautpflegenden und/oder barrierestärkenden Eigenschaften, mit einem Gehalt an gegen Bakterien, Mykota, Viren, Parasiten und Protozoen wirkenden (ω-1)-Hydroxyfettsäuren, insbesondere (ω-1)-Hydroxysäuren mit 10 bis 20 Kohlenstoffatomen, und/oder deren Salzen und/oder einem Gehalt an unverzweigten Fettsäuren mit einer Kettenlänge von C₁₈ bis C₂₈ oder deren Salzen, wobei die (ω-1)-Hydroxysäuren und/oder die genannten Fettsäuren sowohl einzeln als auch im Gemisch vorliegen können.

Die Aufgaben der vorliegenden Erfindung werden überraschend auch gelöst durch die Verwendung von Insektenwachshydrolysaten und/oder den nach dem erfindungsgemäßen Verfahren erhaltenen oder erhältlichen Insektenwachsfraktionen, insbesondere solchen, die einen hohen Gehalt an (ω-1)-Hydroxysäuren und/oder unverzweigten Fettsäuren mit einer Kettenlänge von C₁₈ bis C₂₆ aufweisen.

Bevorzugt werden kosmetisch verträgliche Salze, insbesondere Alkali- oder Ammoniumsalze, beispielsweise Natriumsalze oder Kaliumsalze.

Erfindungsgemäß können die Fettsäuren und/oder die (ω-1)-Hydroxyfettsäuren und/oder die Insektenwachshydrolysate und/oder die Insektenwachsfraktionen von zwei, mehreren, aber auch einer größeren Anzahl von Verbindungen gebildet werden und diese Verbindungen in unterschiedlichen Gewichtsmengen enthalten. So können aus natürlichen Quellen stammende Gemische aufgrund von Homologen oder Strukturisomeren eine größere Zahl aufweisen, synthetisierte Säuren können dagegen aus jeweils einer Verbindung bestehen. Die erfindungsgemäßen, antimikrobiell wirksamen (ω-1)-Hydroxysäuren können geradkettig oder verzweigt sein. Erfindungsgemäß soll mindestens eine der genannten Fettsäuren und/oder (ω-1)- Hydroxysäuren verwendet werden oder in der Zubereitung enthalten sein.

Es wurde überraschenderweise gefunden, daß die erfindungsgemäßen Wirkstoffe sowie die damit erhältlichen kosmetischen, dermatologischen oder medizinischen Mittel eine ausgeprägte gegen Bakterien, Mykota, Viren, Parasiten und Protozoen gerichtete Wirkung besitzen und sich dabei gleichzeitig durch eine ausgesprochen gute Hautverträglichkeit auszeichnen.

Ebenfalls überraschend zeigte sich, daß die erfindungsgemäßen Fettsäuren und/oder (ω-1)-Hydroxysäuren sowie die damit erhältlichen kosmetischen, dermatologischen oder medizinischen Mittel eine sehr hohe Pflegewirkung der Haut zeigen, die Haut geschmeidig haften, das Regenerationsvermögen der Haut unterstützen und die Barriereeigenschaften der Haut erhalten und sogar wiederherstellen. Zubereitungen im Sinne der vorliegenden Erfindung eignen sich daher hervorragend zur Pflege der gesunden, aber auch der kranken Haut.

Durch die Hydroxygruppe enthalten die erfindungsgemäßen Hydroxysäuren mindestens ein asymmetrisches Kohlenstoffatom, und sie können als Racemat, Diastereoisomere oder in der optisch aktiven D- oder L-Form vorliegen. Bevorzugt werden entweder die Verwendung der D-Form oder die der L-Form sowie Zubereitungen, die entweder die D-Form oder die L-Form enthalten.

Bevorzugte erfindungsgemäße antimikrobiell wirksame (ω-1)-Hydroxysäuren weisen einen hohen Gehalt an (ω-1)-Hydroxysäuren mit 14 bis 20 Kohlenstoffatomen oder deren Salzen auf, insbesondere aber einen hohen Gehalt an (ω-1)-Hydroxysäuren mit 16 bis 18 Kohlenstoffatomen oder deren Salzen.

Besonders bevorzugt sind (ω-1)-Hydroxysäuren mit einem hohen Gehalt an den folgenden (ω-1)-Hydroxysäuren und ihre Salzen:
(ω-1)-Hydroxy-hexadecansäure [(ω-1)-Hydroxy-palmitinsäure]
(ω-1)-Hydroxy-tetradecansäure [(ω-1)-Hydroxy-myristinsäure]
(ω-1)-Hydroxy-octadecansäure [(ω-1)-Hydroxy-stearinsäure]
   oder
(ω-1)-Hydroxy-16-methylheptadecansäure.

Ganz besonders bevorzugt werden die optischen Isomere der vorstehend genannten Hydroxyfettsäuren und Gemische, d.h. jeweils der D- oder L-Form, insbesondere aber der D-Form.

Das für (ω-1)-Hydroxysäuren und/oder unverzweigten Fettsäuren Gesagte gilt selbstverständlich entsprechend auch für die erfindungsgemäßen Insektenwachshydrolysate und -fraktionen, insbesondere für solche, die einen hohen Gehalt an (ω-1)-Hydroxysäuren und/oder unverzweigten Fettsäuren mit einer Kettenlänge von C₁₈ bis C₂₆ aufweisen.

Erfindungsgemäß können vorteilhaft aus Insektenwachsen gewonnene (ω-1)-Hydroxysäuren eingesetzt werden, z.B. auch gemäß vorstehendem Isolierungsverfahren erhältliche, aus Insektenwachsen gewonnene (ω-1)-Hydroxy-Fettsäuregemische. Sie fallen vorzugsweise als D-Enantiomeren-Gemisch an. Auch die nach bekannten Verfahren daraus erhältlichen Einzelverbindungen sind bevorzugt.

Besonders bevorzugt werden solche Insektenwachshydrolysate bzw. Insektenwachsfraktionen, in denen der Gewichtsanteil der (ω-1)-Hydroxysäuren bezogen auf das Gesamtgewicht der Mischungen höher ist als in bekannten Insektenwachssäuregemischen. Solche Fraktionen, in denen der (ω-1)-Hydroxysäure-Anteil angereichert ist, sind beispielsweise nach den vorstehenden Isolierungs- und Reinigungsverfahren erhältlich.

Es ist erfindungsgemäß möglich, daß beispielsweise aus Insektenwachssäuregemischen gewonnene erfindungsgemäße Insektenwachshydrolysate bzw. Insektenwachsfraktionen Anteile an (nicht hydroxylierten) Fettsäuren enthalten.

Bevorzugt werden solche Mischungen, in denen dieser Fettsäureanteil nicht zur antimikrobiellen Wirkung beiträgt und besonders bevorzugt werden solche Mischungen, in denen ein möglicher Anteil an iso-Fettsäuren, insbesondere an iso-C₁₄- und iso-C₁₆-Carbonsäuren, nicht zur antimikrobiellen Wirkung beiträgt. Solche Fettsäuren besitzen keine oder nur sehr geringe antimikrobielle Eigenschaften.

Bevorzugt werden Mischungen, in denen dieser Fettsäureanteil reich ist an unverzweigten Fettsäuren mit einer Kettenlänge zwischen C₁₈ und C₂₆, insbesondere zwischen C₂₀ und C₂₄ mit einem Verteilungsmaximum bei C₂₄, besonders bevorzugt solche Mischungen, in denen der Anteil an unverzweigten Fettsäuren mit einer Kettenlänge zwischen C₁₈ und C₂₆, insbesondere zwischen C₂₀ und C₂₄ mit einem Verteilungsmaximum bei C₂₄ zur hautpflegenden und regenerativen Wirkung beiträgt bzw. die hautpflegende und regenerative Wirkung der Mischung bedingt.

Es ist vorteilhaft im Sinne der vorliegenden Erfindung, Insektenwachse und/oder Insektenwachsgemische und/oder Insektenwachshydrolysate und/oder Insektenwachsfraktionen mit einem Fettsäureanteil, der reich ist an unverzweigten Fettsäuren mit einer Kettenlänge zwischen C₁₈ und C₂₆, insbesondere zwischen C₂₀ und C₂₄ mit einem Verteilungsmaximum bei C₂₄, als kosmetisches oder dermatologisches Mittel in Form einer Hautpflegezubereitung zu verwenden.

Besonders bevorzugt werden solche Mischungen, in denen der Gewichtsanteil an (nicht hydroxylierten) Fettsäuren bezogen auf das Gesamtgewicht der Mischungen höher ist als in bekannten Insektenwachssäuregemischen. Solche Fraktionen, in denen der Fettsäureanteil angereichert ist, sind beispielsweise nach den vorstehenden Isolierungs- und Reinigungsverfahren erhältlich.

Vorzugsweise soll der Gehalt des erfindungsgemäßen kosmetischen, dermatologischen oder medizinischen Mittels an unverzweigten Fettsäuren mit einer Kettenlänge zwischen C₁₈ und C₂₆ 0,01 bis 10 Gew.-%, bezogen auf die Gesamt-Zusammensetzung des Mittels oder der Zubereitung, betragen, insbesondere aber 0,1 bis 1 Gew.-%, besonders bevorzugt 0,25 bis 0,75 Gew.-%. Bei Verwendung von Insektenwachshydrolysaten und/oder Insektenwachsfraktionen, welche beispielsweise nach dem vorstehenden Isolierungsverfahren erhältlich sind, entspricht dies etwa einem Gehalt von 0,5 bis 65 Gew.-%, insbesondere 2 bis 35 Gew.-% und besonders bevorzugt 2,5 bis 20 Gew.-% an der jeweiligen Insektenwachsfraktion.

Die erfindungsgemäßen, antimikrobiell wirksamen (ω-1)-Hydroxysäuren sind bekannt oder nach bekannten Verfahren erhältlich. In der Synthese anfallende Racemate lassen sich in üblichen Verfahren in die Isomere aufspalten.

Es stellte sich überraschenderweise heraus, daß (ω-1)-Hydroxysäuren eine hohe antimikrobielle Wirksamkeit gegenüber Hautbakterien besitzen.

Es wurde gefunden, und auch darin liegt die Lösung der Aufgabe gemäß der Erfindung, daß man unter Verwendung der üblichen kosmetischen, dermatologischen oder medizinischen Trägerstoffe zu antimikrobiell wirksamen kosmetischen, dermatologischen oder medizinischen Mitteln mit sehr guten antimikrobiellen Eigenschaften gelangt, wenn man derartigen Mitteln geringe Mengen von den erfindungsgemäßen antimikrobiell wirkenden (ω-1)-Hydroxyfettsäuren zusetzt. Da Insektenwachssäuren einen sehr geringen Eigengeruch aufweisen, sind sie insbesondere in Kosmetika oder Dermatika sehr gut einarbeitbar.

Gegenstand der Erfindung ist somit auch ein antimikrobiell wirksames kosmetisches, dermatologisches oder medizinisches Mittel auf Basis üblicher kosmetischer, dermatologischer oder medizinischer Trägerstoffe sowie ein oder mehrerer Wirkstoffe, das dadurch gekennzeichnet ist, daß es als Wirkstoff die genannten antimikrobiell wirksamen (ω-1)-Hydroxysäuren enthält.

Das für (ω-1)-Hydroxysäuren Gesagte gilt selbstverständlich entsprechend auch für die erfindungsgemäßen Insektenwachshydrolysate und -fraktionen, insbesondere für solche, die einen hohen Gehalt an (ω-1)-Hydroxysäuren und gewünschtenfalls unverzweigten Fettsäuren mit einer Kettenlänge von C₁₈ bis C₂₆ aufweisen.

Vorzugsweise soll der Gehalt des erfindungsgemäßen antimikrobiell wirksamen kosmetischen, dermatologischen oder medizinischen Mittels an antimikrobiell wirksamen (ω-1)-Hydroxyfettsäuren 0,01 bis 10 Gew.-%, bezogen auf die Gesamt-Zusammensetzung des Mittels oder der Zubereitung, betragen, insbesondere aber 0,1 bis 1 Gew.-%, besonders bevorzugt aber 0,25 bis 0,75 Gew.-%. Bei Verwendung von Insektenwachshydrolysaten und/oder Insektenwachsfraktionen, insbesondere solchen, die einen hohen Gehalt an (ω-1)-Hydroxysäuren mit 10 bis 20 Kohlenstoffatomen oder deren Salzen aufweisen und die beispielsweise nach dem vorstehenden Isolierungsverfahren erhältlich sind, entspricht dies etwa einem Gehalt von 0,5 bis 65 Gew.-%, insbesondere 2 bis 35 Gew.-% und besonders bevorzugt 2,5 bis 20 Gew.-% an der jeweiligen Insektenwachsfraktion.

Das antimikrobiell wirksame kosmetische, dermatologische oder medizinische Mittel gemäß der Erfindung kann beispielsweise in Form eines Deodorants vorliegen. Es kann als aus Aerosolbehältern, Quetschflaschen oder durch eine Pumpvorrichtung versprühbares Präparat vorliegen oder in Form einer mittels Roll-on-Vorrichtungen auftragbaren flüssigen Zusammensetzung, jedoch auch in Form einer aus normalen Flaschen und Behältern auftragbaren W/O- oder O/W-Emulsion, z.B. einer Creme oder Lotion.

Kosmetische Desodorantien können neben den obengenannten auch in Form von desodorierenden Tinkturen, desodorierenden Intimreinigungsmitteln, desodorierenden Shampoos, Deo-Seifen, desodorierenden Dusch- oder Badezubereitungen, desodorierenden Pudern oder desodorierenden Pudersprays vorliegen. Es können aber auch übliche Deo-Stift-Grundmassen als Träger für feste Zubereitungen und Stifte dienen.

Als übliche kosmetische, dermatologische oder medizinische Trägerstoffe zur Herstellung der desodorierenden Mittel gemäß der Erfindung können neben Wasser, Ethanol und Isopropanol, Glycerin, und Propylenglykol hautpflegende Fell- oder fettähnliche Stoffe, wie Partialglyceride von Fettsäuregemischen, Ölsäuredecylester, Cetylalkohol, Cetylstearylalkohol und 2-Octyldodecanol, in den für solche Präparate üblichen Mengenverhältnissen eingesetzt werden sowie schleimbildende Stoffe und Verdickungsmittel, wie Methylcellulose, Polyacrylsäure, Polyvinylpyrrolidon, daneben aber auch in kleinen Mengen cyclische Silikonöle (Polydimethylsiloxane) sowie flüssige Polymethylphenylsiloxane niedriger Viskosität.

Als Treibmittel für aus Aerosolbehältem versprühbare kosmetische und/oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Als Emulgatoren zur Herstellung der erfindungsgemäßen kosmetischen, dermatologischen oder medizinischen Zubereitungen, die vorzugsweise als flüssige Zubereitungen mittels einer Roll-on-Vorrichtung auf die gewünschten Hautbereiche aufgetragen werden sollen und die in den Mitteln in geringer Menge (z.B. 2 bis 5 Gew.-%, bezogen auf die Gesamt-Zusammensetzung) verwendet werden können, haben sich nichtionogene Typen, wie Polyoxyethylen-Fettalkoholether, z.B. Cetostearylalkoholpolyethylenglykolether mit 12 bzw. 20 angelagerten Ethylenoxid-Einheiten pro Molekül Cetostearylalkohol, sowie Sorbitanester und Sorbitanester-Ethylenoxid-Verbindungen (z.B. Sorbitanmonostearat und Polyoxyethylensorbitanmonostearat) als auch langkettige höhermolekulare wachsartige Polyglykolether als geeignet erwiesen.

Zusätzlich zu den genannten Bestandteilen können den kosmetischen, dermatologischen oder medizinischen Zubereitungen gemäß der Erfindung, deren pH-Wert 2 bis 12 betragen kann, vorzugsweise aber - z.B. durch übliche Puffergemische - auf 5 bis 7,5, insbesondere 6 bis 7,5, eingestellt wird, kleine Mengen Parfüm, Farbstoffe, Suspendiermittel, Puffergemische oder andere übliche kosmetische, dermatologische oder medizinische Grundstoffe, wie Triethanolamin oder Harnstoff, beigemischt werden. Für Deo-Stifte und Deo-Seife wird ein pH-Bereich von 8,5 bis 9,8 bevorzugt. Insgesamt wird ein pH-Bereich von 5 bis 10 bevorzugt.

Die erfindungsgemäßen Insektenwachshydrolysate und/oder Insektenwachsfraktionen, die vorzugsweise zwischen 10 und 90 Gew.-% (ω-1)-Hydroxysäuren mit 10 bis 20 Kohlenstoffatomen und zwischen 90 und 10 Gew.-% langkettige, unverzweigte Fettsäuren mit Kettenlängen von 18 bis 26 Kohlenstoffatomen enthalten, werden im Sinne der vorliegenden Erfindung vorteilhaft in antimikrobiellen und/oder hautpflegenden kosmetischen, dermatologischen oder medizinischen Mitteln eingesetzt.

Die erfindungsgemäßen kosmetischen oder dermatologischen Mittel können wie üblich zusammengesetzt sein und zur Behandlung der Haut und/oder der Haare und als Schminkprodukt in der dekorativen Kosmetik dienen. Entsprechend können die erfindungsgemäßen Zubereitungen, je nach ihrem Aufbau, beispielsweise verwendet werden als Hautschutzcrème, Reinigungsmilch, Sonnenschutzlotion, Nährcrème, Tages- oder Nachtcrème usw. Es ist gegebenenfalls möglich und vorteilhaft, die erfindungsgemäßen Zubereitungen als Grundlage für pharmazeutische Formulierungen zu verwenden.

Günstig sind auch solche kosmetischen und dermatologischen Zubereitungen, die in der Form eines Sonnenschutzmittels vorliegen. Vorzugsweise enthalten diese neben einem oder mehreren erfindungsgemäßen Wirkstoffen mindestens eine UV-A-Filtersubstanz und/oder mindestens eine UV-B-Filtersubstanz und/oder mindestens ein anorganisches Pigment.

Es ist aber auch vorteilhaft im Sinne der vorliegenden Erfindung, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden z.B. in Tagescrèmes gewöhnlich UV-A- bzw. UV-B-Filtersubstanzen eingearbeitet.

Die jeweils einzusetzenden Mengen an kosmetischen, dermatologischen oder medizinischen Trägerstoffen und Parfüm können in Abhängigkeit von der Art des jeweiligen Produktes vom Fachmann durch einfaches Ausprobieren leicht ermittelt werden.

Zur Parfümierung sind insbesondere solche Substanzen und Parfümöle geeignet, die stabil sind, die Haut nicht reizen und bereits als solche antibakterielle bzw. bakteriostatische Eigenschaften besitzen.

Die Herstellung der kosmetischen, dermatologischen oder medizinischen Zubereitungen erfolgt abgesehen von speziellen Zubereitungen, die in den Beispielen jeweils gesondert vermerkt sind, in üblicher Weise, zumeist durch einfaches Vermischen unter Rühren, gegebenenfalls unter leichter Erwärmung. Sie bietet keine Schwierigkeiten. Für Emulsionen werden Fettphase und die Wasserphase z.B. separat gegebenenfalls unter Erwärmen hergestellt und dann emulgiert.

Ansonsten sind die üblichen Maßregeln für das Zusammenstellen von kosmetischen, dermatologischen oder medizinischen Formulierungen zu beachten, die dem Fachmann geläufig sind.

Die erfindungsgemäßen Wirkstoffe können als solche oder in gelöster Form oder als Suspension (z.B. als wäßrige, alkoholische oder alkoholisch-wäßrige Lösung oder Suspension) den übrigen Bestandteilen der Formulierungen zugesetzt werden.

Insbesondere können die erfindungsgemäßen kosmetischen, dermatologischen oder medizinischen Zubereitungen auch Antioxidantien enthalten. Erfindungsgemäß können als günstige Antioxidantien alle für kosmetische, dermatologische und/oder medizinische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure sowie andere unglycosylierte sowie ein oder mehrfach glycosylierte Flavon- und Isoflavon-Derivate, Furfurylidenglucitol, Camosin, Butylhydroxytoluol, Butylhydroxyanisol, Nordihydroguajakharzsäure, Nordihydroguajaret-säure, Trihydroxybutyrophenon, Harnsäure und deren Derivate, Mannose und deren Derivate, Zink und dessen Derivate (z.B. ZnO, ZnSO₄) Seien und dessen Derivate (z.B. Selenmethionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den erfindungsgemäßen Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Vorteilhaft ist auch, einen oder mehrere erfindungsgemäße Wirkstoffe in sogenannte Pudersprays einzuarbeiten. Sollen die erfindungsgemäßen Kombinationen in Pudersprays eingearbeitet werden, so können die Suspensionsgrundlagen dafür vorteilhaft gewählt werden aus der Gruppe Aerosil, Kieselgur, Kaolin, Talkum, modifizierte Stärke, Titandioxid, Seidenpulver, Nylonpulver, Polyethylenpulver und verwandten Stoffen.

Die Erfindung ist aber selbstverständlich nicht auf die genannten Zubereitungen und Mittel, Hilfsstoffe und Trägersubstanzen beschränkt.

Alle Prozentangaben oder Teile beziehen sich, soweit nicht anders angegeben, auf das Gewicht, insbesondere auf das Gesamtgewicht der Zubereitungen.

Die Erfindung wird anschließend anhand von Beispielen näher erläutert. Die gemäß den folgenden Beispielen hergestellten Präparate zeigten in jedem Falle neben einer ausgezeichneten Haut- und Schleimhautverträglichkeit auch hautpflegende und regenerative Eigenschaften sowie bei bestimmungsmäßer Anwendung eine sehr gute und lang anhaftende antimikrobielle Wirksamkeit.

### Konservierungshelfer

Gegenstand der Erfindung sind auch die Verwendung von (ω-1)-Hydroxysäuren und/oder Insektenwachshydrolysaten bzw. Insektenwachsfraktionen aus Insektenwachsen, insbesondere solchen, die einen hohen Gehalt an (ω-1)-Hydroxysäuren aufweisen, als Konservierungshelfer und insbesondere kosmetische, dermatologische oder medizinische Zubereitungen, die diese enthalten.

Konservierungshelfer sind Substanzen oder Stoffe, die, in entsprechender Konzentration eingesetzt, Nahrungs- oder Genußmittel, pharmazeutische, kosmetische oder auch chemisch-technische Zubereitungen vor insbesondere mikrobiell bedingten nachteiligen Veränderungen schützen sollen. An einen Konservierungshelfer sollten grundsätzlich nachfolgende Forderungen gestellt werden: er muß ausreichend antimikrobiell wirksam, technologisch anwendbar und gesundheitlich unbedenklich sein. Dabei muß auch die fertige kosmetische, dermatologische oder medizinische Zubereitung, das Handelsprodukt, das Kriterium der gesundheitlichen Unbedenklichkeit erfüllen. Es ist zu berücksichtigen, daß Mikroorganismen in kosmetischen, dermatologischen oder medizinischen Zubereitungen primär produktionsbedingt vorhanden sein oder sekundär durch den Verbraucher in das Mittel gelangen können. Aus diesem Grunde muß der Hersteller des kosmetischen, dermatologischen oder medizinischen Mittels dafür sorgen, daß das Fertigprodukt auch über den gesamten Verbrauchszeitraum sicher ist.

Aufgabe der Erfindung ist es, als Konservierungshelfer Stoffe bereitzustellen und kosmetische, dermatologische oder medizinische Mittel und Zubereitungen mit Konservierungshelfern zu schaffen, die die Nachteile des Standes der Technik nicht aufweisen und insbesondere einen im Hinblick auf die Wirksamkeit, Hautschonung und Verträglichkeit verbesserten Wirkstoff zur Verfügung zu stellen sowie kosmetische, dermatologische oder medizinische Zubereitungen, die diesen Konservierungshelfer enthalten.

Überraschenderweise werden die Aufgaben der vorliegenden Erfindung gelöst durch die Verwendung von (ω-1)-Hydroxyfettsäuren, insbesondere (ω-1)-Hydroxysäuren mit 10 bis 20 Kohlenstoffatomen, und/oder deren Salzen als Konservierungshelfer in kosmetischen, dermatologischen oder medizinischen Zubereitungen, wobei die (ω-1)-Hydroxysäuren sowohl einzeln als auch im Gemisch vorliegen können, sowie durch kosmetische, dermatologische oder medizinische Zubereitungen mit einem Gehalt an als Konservierungshelfer wirkenden (ω-1)-Hydroxyfettsäuren, insbesondere (ω-1)-Hydroxysäuren mit 10 bis 20 Kohlenstoffatomen, und/oder deren Salzen, wobei die (ω-1)-Hydroxysäuren sowohl einzeln als auch im Gemisch vorliegen können.

Die Aufgaben der vorliegenden Erfindung werden überraschend auch gelöst durch die Verwendung von Insektenwachshydrolysaten und/oder den nach dem erfindungsgemäßen Verfahren erhaltenen oder erhältlichen Insektenwachsfraktionen, insbesondere solchen, die einen hohen Gehalt an (ω-1)-Hydroxysäuren aufweisen.

Bevorzugt werden kosmetisch verträgliche Salze der (ω-1)-Hydroxysäuren, insbesondere Alkali- oder Ammoniumsalze, beispielsweise Natrium- oder Kaliumsalze, verwendet.

Erfindungsgemäß können die Fettsäuren und/oder die (ω-1)-Hydroxyfettsäuren und/oder die Insektenwachshydrolysate und/oder die Insektenwachsfraktionen von zwei, mehreren, aber auch einer größeren Anzahl von Verbindungen gebildet werden und diese Verbindungen in unterschiedlichen Gewichtsmengen enthalten. So können aus natürlichen Quellen stammende Gemische aufgrund von Homologen oder Strukturisomeren eine größere Zahl aufweisen, synthetisierte Säuren können dagegen aus jeweils einer Verbindung bestehen. Die erfindungsgemäß als Konservierungshelfer wirksamen (ω-1)-Hydroxysäuren können geradkettig oder verzweigt sein. Erfindungsgemäß soll mindestens eine der genannten (ω-1)-Hydroxysäuren verwendet werden oder in der Zubereitung enthalten sein.

Es wurde überraschenderweise gefunden, daß die erfindungsgemäßen (ω-1)-Hydroxysäuren, ihre Salze und/oder Insektenwachshydrolysate und/oder Insektenwachsfraktionen hervorragende Konservierungshelfer darstellen.

Durch die Hydroxygruppe erhalten die Hydroxysäuren mindestens ein asymmetrisches Kohlenstoffatom, und sie können als Racemat, Diastereoisomere oder in der optisch aktiven D- oder L-Form vorliegen. Bevorzugt werden entweder die Verwendung der D-Form oder die der L-Form sowie Zubereitungen, die entweder die D-Form oder die L-Form enthalten.

Bevorzugte erfindungsgemäße als Konservierungshelfer wirkende (ω-1)-Hydroxysäuren weisen einen hohen Gehalt an (ω-1)-Hydroxysäuren mit 14 bis 20 Kohlenstoffatomen oder deren Salzen auf, insbesondere aber einen hohen Gehalt an (ω-1)-Hydroxysäuren mit 16 bis 18 Kohlenstoffatomen oder deren Salzen.

Besonders bevorzugt sind die folgenden Hydroxysäuren und ihre Salze:
(ω-1)-Hydroxy-hexadecansäure [(ω-1)-Hydroxypalmitinsäure]
(ω-1)-Hydroxy-octadecansäure [(ω-1)-Hydroxystearinsäure]
(ω-1)-Hydroxy-16-methylheptadecansäure
(ω-1)-Hydroxy-dodecansäure [(ω-1)-Hydroxylaurinsäure]
   und/oder
(ω-1)-Hydroxy-tetradecansäure [(ω-1)-Hydroxymyristinsäure].

Ganz besonders bevorzugt werden die optischen Isomere der vorstehend genannten Hydroxyfettsäuren und Gemische, d.h. jeweils der D- und L-Form, insbesondere aber der D-Form.

Das für (ω-1)-Hydroxysäuren Gesagte gilt selbstverständlich entsprechend auch für die erfindungsgemäßen Insektenwachshydrolysate und -fraktionen, insbesondere für solche, die einen hohen Gehalt an (ω-1)-Hydroxysäuren und gewünschtenfalls unverzweigten Fettsäuren mit einer Keffenlänge von C₁₈ bis C₂₆ aufweisen.

Erfindungsgemäß können vorteilhaft aus Insektenwachs gewonnenene (ω-1)-Hydroxysäuren eingesetzt werden, z.B. auch gemäß vorstehendem Isolierungsverfahren erhältliche (ω-1)-Hydroxysäuregemische, die aus Insektenwachssäurehydrolysaten gewonnen werden. Sie fallen vorzugsweise als D-Enantiomeren-Gemisch an. Auch die nach bekannten Verfahren daraus erhältlichen Einzelverbindungen sind bevorzugt.

Es wurde überraschenderweise gefunden, daß die erfindungsgemäßen Wirkstoffe als Konservierungshelfer wirken und daß sogar im sauren pH-Bereich, insbesondere im Bereich von pH 5 - 6,5, gegenüber gramnegativen Organismen eine ebenso hohe antimikrobielle Wirksamkeit vorhanden war, wie bei pH 7,5. Diese Eigenschaft ist für Konservierungshelfer sehr vorteilhaft.

Die beiden optisch aktiven Enantiomeren sind noch wirksamer als das Racemat. Dieses Ergebnis wurde zusätzlich dadurch bestätigt, daß die erneute Vermischung der Enantiomeren wieder zu der Aktivität führte, wie sie bei dem Racemat beobachtet wurde.

Als Konservierungshelfer können die (ω-1)-Hydroxysäuren gemäß der Erfindung in kosmetischen, dermatologischen oder medizinischen Zubereitungen wie kosmetischen, dermatologischen oder medizinischen Rohstoffen, kosmetischen, dermatologischen oder medizinischen Vorprodukten, und insbesondere in kosmetischen, dermatologischen oder medizinischen Mitteln verwendet werden.

Kosmetische, dermatologische oder medizinische Zubereitungen im Sinne der vorliegenden Erfindung sind daher auch kosmetische, dermatologische oder medizinische Rohstoffe, kosmetische, dermatologische oder medizinische Vorprodukte und kosmetische, dermatologische oder medizinische Mittel mit den erfindungsgemäßen, als Konservierungshelfer wirkenden (ω-1)-Hydroxysäuren. Geeignet sind alle üblichen kosmetischen, dermatologischen oder medizinischen Zubereitungen bzw. Mittel oder Kosmetika.

Das für (ω-1)-Hydroxysäuren Gesagte gilt selbstverständlich entsprechend auch für die erfindungsgemäßen Insektenwachshydrolysate und -fraktionen, insbesondere für solche, die einen hohen Gehalt an (ω-1)-Hydroxysäuren und gewünschtenfalls unverzweigten Fettsäuren mit einer Keffenlänge von C₁₈ bis C₂₆ aufweisen.

Die erfindungsgemäßen kosmetischen oder dermatologischen Mittel können wie üblich zusammengesetzt sein und zur Behandlung der Haut und/oder der Haare und als Schminkprodukt in der dekorativen Kosmetik dienen. Entsprechend können die erfindungsgemäßen Zubereitungen, je nach ihrem Aufbau, beispielsweise verwendet werden als Hautschutzcrème, Reinigungsmilch, Sonnenschutzlotion, Nährcrème, Tages- oder Nachtcrème usw. Es ist möglich und vorteilhaft, die erfindungsgemäßen Zubereitungen als Grundlage für pharmazeutische Formulierungen zu verwenden.

Günstig sind auch solche kosmetischen und dermatologischen Zubereitungen, die in der Form eines Sonnenschutzmittels vorliegen. Vorzugsweise enthalten diese neben einem oder mehreren erfindungsgemäßen Wirkstoffen mindestens eine UV-A-Filtersubstanz und/oder mindestens eine UV-B-Filtersubstanz und/oder mindestens ein anorganisches Pigment.

Es ist aber auch vorteilhaft im Sinne der vorliegenden Erfindung, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden z.B. in Tagescrèmes gewöhnlich UV-A- bzw. UV-B-Filtersubstanzen eingearbeitet.

Vorzugsweise soll der Gehalt des erfindungsgemäßen kosmetischen, dermatologischen oder medizinischen Mittels an konservierend wirkenden (ω-1)-Hydroxyfettsäuren 0,01 bis 10 Gew.-%, bezogen auf die Gesamt-Zusammensetzung des Mittels oder der Zubereitung, betragen, insbesondere aber 0,1 bis 1 Gew.-%, besonders bevorzugt aber 0,25 bis 0,75 Gew.-%. Bei Verwendung von Insektenwachshydrolysaten und/oder Insektenwachsfraktionen, insbesondere solchen, die einen hohen Gehalt an (ω-1)-Hydroxysäuren mit 10 bis 20 Kohlenstoffatomen oder deren Salzen aufweisen und die beispielsweise nach dem vorstehenden Isolierungsverfahren erhältlich sind, entspricht dies etwa einem Gehalt von 0,5 bis 65 Gew.-%, insbesondere 2 bis 35 Gew.-% und besonders bevorzugt 2,5 bis 20 Gew.-% an der jeweiligen Insektenwachsfraktion.

Als zweckmäßig hat es sich erwiesen, den pH-Wert der Zubereitungen und der kosmetischen, dermatologischen oder medizinischen Formulierung im Bereich von 2 bis 12 einzustellen, vorzugsweise von 4,5 bis 9, insbesondere aber im Bereich von pH 5 bis pH 7.

Gegenstand der Erfindung sind auch die kosmetischen, dermatologischen oder medizinischen Zubereitungen, insbesondere kosmetische, dermatologische oder medizinische Mittel auf Basis üblicher kosmetischer, dermatologischer oder medizinischer Trägerstoffe sowie ein oder mehrerer Wirkstoffe, wobei der Wirkstoff eine erfindungsgemäße als Konservierungshelfer wirkende (ω-1)-Hydroxysäure bzw. eine die erfindungsgemäße (ω-1)-Hydroxysäure enthaltende Insektenwachsfraktion ist.

Geeignet sind beispielsweise die bekannten kosmetischen, dermatologischen oder medizinischen Zubereitungen wie O/W-Emulsionen, W/O-Emulsionen, Cremes, Lotionen, Milchen, Salben, Fettsalben, Gele, Tinkturen, Solubilisate, Lösungen, Suspensionen, ölige Zubereitungen oder feste, beispielsweise stiftförmige Zubereitungen, Puder oder Sprays in allen Formen.

Die Kosmetika oder Dermatika können in bekannter Weise Trägerstoffe, Hilfsstoffe und Zusatzstoffe enthalten. Es sind z.B. wasserbindende Stoffe, Verdicker, Füllstoffe, Parfüm, Farbstoffe, Tenside, Lichtschutzmittel, Öle, Fette, Wachse, Sonnenschutzmittel, UV-A-Blocker, UV-B-Blocker, Vitamine, Proteine, Insektenrepellentien, Stabilisatoren, Antioxidantien, Konservierungsmittel, Alkohole, keratolytisch oder proteolytisch wirksame Substanzen, Wasser und/oder Salze, beispielsweise Puffersalze oder Puffergemische zur pH-Einstellung.

Kosmetische, dermatologische oder medizinische Rohstoffe und Vorprodukte sind beispielsweise Einzelverbindungen, Mischungen oder Konzentrate von kosmetischen, dermatologischen oder medizinischen Trägerstoffen, Hilfsstoffen oder Wirkstoffen und unterscheiden sich daher nicht wesentlich von kosmetischen, dermatologischen oder medizinischen Mitteln, so daß auf diese bezogene Angaben sich gleichermaßen auf solche Rohstoffe oder Vorprodukte beziehen.

Bevorzugte erfindungsgemäße Kosmetika oder Dermatika sind wasserhaltige Mittel wie WAS-Zubereitungen, z.B. Lösungen, Suspensionen, Dispersionen oder Gele, insbesondere auf Wasserbasis oder Wasser-Alkohol-Basis.

Besonders bevorzugt sind Badepräparate, Schaumbäder, Duschgele und Duschöle sowie Shampoos.

Die erfindungsgemäßen Konservierungshelfer zeichnen sich durch eine gute Hautverträglichkeit und eine gute Wirkung aus. Auch die hergestellten Präparate besitzen gute Wirkung und Verträglichkeit. Eine sehr gute Hautverträglichkeit zeigt sich insbesondere dann, wenn der Wirkstoff eine eine erfindungsgemäße (ω-1)-Hydroxysäure enthaltende Insektenwachsfraktion ist, die gleichzeitig einen Gehalt an unverzweigten Fettsäuren mit einer Kettenlänge von C₁₈ bis C₂₆ oder deren Salzen aufweist, wobei die (ω-1)-Hydroxysäuren und/oder die genannten Fettsäuren sowohl einzeln als auch im Gemisch vorliegen können.

Zur Parfümierung sind insbesondere solche Substanzen und Parfümöle geeignet, die stabil sind und die Haut nicht reizen.

Die Erfindung wird in den Beispielen erläutert. Die Erfindung ist aber nicht auf die genannten Zubereitungen und Mittel, Hilfsstoffe und Trägersubstanzen beschränkt.

Die Herstellung der kosmetischen, dermatologischen oder medizinischen Mittel erfolgt abgesehen von speziellen Zubereitungen, die in den Beispielen jeweils gesondert vermerkt sind, in üblicher Weise, zumeist durch einfaches Vermischen unter Rühren, gegebenenfalls unter leichter Erwärmung. Sie bietet keine Schwierigkeiten. Für Emulsionen werden die Fettphase und die Wasserphase z.B. separat gegebenenfalls unter Erwärmen hergestellt und dann emulgiert.

Die erfindungsgemäßen Wirkstoffe können als solche oder in gelöster Form oder als Suspension (z.B. als wäßrige, alkoholische oder alkoholisch-wäßrige Lösung oder Suspension) den übrigen Bestandteilen der Formulierungen zugesetzt werden.

Zubereitungen, in denen erfindungsgemäße Wirkstoffe als Konservierungshelfer enthalten sind, können topische Zubereitungen sein, beispielsweise kosmetische und dermatologische topische Zubereitungen oder aber auch übliche Arzneimittel-Darreichungsformen.

Die konservierenden Wirkstoffe gemäß der Erfindung können vorzugsweise in Zubereitungen eingesetzt werden, die in Form von Granulaten, Kapseln, Pillen, Tabletten, Filmtabletten, Dragees, Sirupen, Emulsionen, Suspensionen, Dispersionen, Aerosolen und Lösungen sowie Flüssigkeiten oder auch als Zäpfen oder Vaginalkugeln vorliegen. Die Arzneimittel-Darreichungsformen können dabei wie üblich zusammengesetzt sein.

Die jeweils einzusetzenden Mengen an kosmetischen, dermatologischen oder medizinischen Trägerstoffen und Parfüm können in Abhängigkeit von der Art des jeweiligen

Produktes vom Fachmann durch einfaches Ausprobieren leicht ermittelt werden. Ansonsten sind die üblichen Maßregeln für das Zusammenstellen von kosmetischen, dermatologischen oder medizinischen Formulierungen zu beachten, die dem Fachmann geläufig sind.

### Ionen-Bindungsvermögen und Ionen-Komplexierungsvermögen

(ω-1)-Hydroxysäuren sowie Insektenwachshydrolysate und -fraktionen, dieselben enthaltend, zeichnen sich überraschenderweise weiter durch ein hohes Komplexierungs- und Bindungsvermögen für Metallionen aus.

Metallionen wie Erdalkali und insbesondere Schwermetallionen können in Kosmetika zu unerwünschten Reaktionen wie Verfärbungen führen. Sie reagieren mit anionischen Bestandteilen von kosmetischen, dermatologischen oder medizinischen Formulierungen zu unlöslichen störenden Komplexen. Die Oxidation von Farb- und Riechstoffen wird durch Spuren von Schwermetallen katalysiert. Die Wirksamkeit von antimikrobiell wirksamen Substanzen wird durch die Anwesenheit dieser Kationen beeinträchtigt. Komplexbildende Substanzen wie beispielsweise Ethylendiamintetraessigsäure bzw. deren Natriumsalz (EDTA) finden daher in der Kosmetik weite Anwendung.

Komplexbildner wie Nitrilotriessigsäure (NTA) oder insbesondere EDTA, besitzen aber den Nachteil, daß sie biologisch schwer abbaubar sind und daher in zunehmenden Maße eine Umweltbelastung darstellen. Es ist bekannt, daß einige alpha-Hydroxycarbonsäuren und auch einige Fettsäuren ein gewisses Ca²⁺-Bindungspotential besitzen. Entsprechende Komplexbildungskonstanten sind für Laktat, Mandelat, Tartrat, Malat und Citrat verfügbar. Diese Säuren bilden aber nicht die für die Verwendung in Kosmetika erforderlichen stabilen Komplexe.

Bekannt ist auch, daß Kupfersalzlösungen mit alpha-Hydroxyfettsäuren Ausfällungen ergeben.

Aufgabe der Erfindung war es, für Kosmetika einen Metallionen bindenden oder komplexierenden Stoff zur Verfügung zu stellen, der die Nachteile des Standes der Technik nicht hat und insbesondere geeignet ist, EDTA in kosmetischen, dermatologischen und medizinischen Formulierungen zu ersetzen.

Diese Aufgabe wird gelöst durch die Verwendung von (ω-1)-Hydroxyfettsäuren und/oder deren Salzen zum Binden oder Komplexieren von Ionen in kosmetischen, dermatologischen oder medizinischen Zubereitungen, wobei die (ω-1)-Hydroxysäuren sowohl einzeln als auch im Gemisch vorliegen können, sowie durch kosmetische, dermatologische oder medizinische Zubereitungen mit einem Gehalt an ionenbindend oder ionenkomplexierend wirkenden (ω-1)-Hydroxyfettsäuren und/oder deren Salzen, wobei die (ω-1)-Hydroxysäuren sowohl einzeln als auch im Gemisch vorliegen können.

Die Aufgaben der vorliegenden Erfindung werden überraschend auch gelöst durch die Verwendung von Insektenwachshydrolysaten und/oder den nach dem erfindungsgemäßen Verfahren erhaltenen oder erhältlichen Insektenwachsfraktionen, insbesondere solchen, die einen hohen Gehalt an (ω-1)-Hydroxysäuren aufweisen.

Für Kosmetika und Dermatika werden kosmetisch und/oder dermatologisch verträgliche Salze, insbesondere Alkali- oder Ammoniumsalze, beispielsweise Natrium- oder Kaliumsalze, bevorzugt.

Erfindungsgemäß können die Fettsäuren und/oder die (ω-1)-Hydroxyfettsäuren und/oder die Insektenwachshydrolysate und/oder die Insektenwachsfraktionen von zwei, mehreren, aber auch einer größeren Anzahl von Verbindungen gebildet werden und diese Verbindungen in unterschiedlichen Gewichtsmengen enthalten. So können aus natürlichen Quellen stammende Fraktionen aufgrund von Homologen oder Strukturisomeren eine größere Zahl aufweisen, synthetisierte Säuren können dagegen aus jeweils einer Verbindung bestehen. Die erfindungsgemäß zum Binden oder Komplexieren wirksamen (ω-1)-Hydroxysäuren können geradkettig oder verzweigt sein. Erfindungsgemäß soll mindestens eine der genannten (ω-1)-Hydroxysäuren verwendet werden oder in der Zubereitung enthalten sein.

Es wurde überraschenderweise gefunden, daß die erfindungsgemäßen (ω-1)-Hydroxysäuren und ihre eine ausgeprägte ionenbindende oder ionenkomplexierende Wirkung besitzen.

Bevorzugte erfindungsgemäße ionenbindende und komplexierende (ω-1)-Hydroxysäuren weisen einen hohen Gehalt an (ω-1)-Hydroxysäuren mit 14 bis 20 Kohlenstoffatomen oder deren Salzen auf, insbesondere aber einen hohen Gehalt an (ω-1)-Hydroxysäuren mit 16 bis 18 Kohlenstoffatomen oder deren Salzen.

Besonders bevorzugt sind die folgenden Hydroxysäuren und ihre Salze:
(ω-1)-Hydroxy-hexadecansäure [(ω-1)-Hydroxypalmitinsäure]
(ω-1)-Hydroxy-octadecansäure [(ω-1)-Hydroxystearinsäure]
(ω-1)-Hydroxy-16-methylheptadecansäure
(ω-1)-Hydroxy-dodecansäure [(ω-1)-Hydroxylaurinsäure]
   und/oder
(ω-1)-Hydroxy-tetradecansäure [(ω-1)-Hydroxymyristinsäure].

Das für (ω-1)-Hydroxysäuren Gesagte gilt selbstverständlich entsprechend auch für die erfindungsgemäßen Insektenwachshydrolysate und -fraktionen, insbesondere für solche, die einen hohen Gehalt an (ω-1)-Hydroxysäuren und gewünschtenfalls unverzweigten Fettsäuren mit einer Kettenlänge von C₁₈ bis C₂₆ aufweisen.

Erfindungsgemäß können vorteilhaft aus Insektenwachs gewonnene (ω-1)-Hydroxysäuren eingesetzt werden, z.B. auch gemäß vorstehendem Isolierungsverfahren erhältliche (ω-1)-Hydroxysäuregemische, die aus Insektenwachshydrolysaten gewonnen werden. Sie fallen vorzugsweise als D-Enantiomeren-Gemisch an. Auch die nach bekannten Verfahren daraus erhältlichen Einzelverbindungen sind bevorzugt. In der Synthese anfallende Racemate lassen sich in üblichen Verfahren in die Isomeren aufspalten.

Vorzugsweise werden erfindungsgemäß Erdalkaliionen und Schwermetallionen gebunden oder komplexiert.

Besonders bevorzugte Ionen sind Ca²⁺, Mg²⁺, Fe²⁺, Fe³⁺, Cu⁺, Cu²⁺, Mn²⁺, Hg⁺ und Hg²⁺.

Die erfindungsgemäßen (ω-1)-Hydroxysäuren haben gegenüber Schwermetallionen, z.B. Fe²⁺ oder Erdalkali-Ionen wie Ca²⁺ eine hohe Bindungsfähigkeit.

Gegenstand der Erfindung sind auch kosmetische, dermatologische oder medizinische Zubereitungen mit den erfindungsgemäßen ionenbindenden und ionenkomplexierenden (ω-1)-Hydroxysäuren. Geeignet sind alle üblichen kosmetischen, dermatologischen oder medizinischen Zubereitungen bzw. Mittel oder Kosmetika.

Die erfindungsgemäßen kosmetischen, dermatologischen oder medizinischen Mittel können wie üblich zusammengesetzt sein und zur Behandlung der Haut und/oder der Haare und als Schminkprodukt in der dekorativen Kosmetik dienen.

Vorzugsweise soll der Gehalt des erfindungsgemäßen kosmetischen, dermatologischen oder medizinischen Mittels an ionenbindenden und ionenkomplexierenden (ω-1)-Hydroxyfettsäuren und/oder Fettsäuregemische 0,01 bis 10 Gew.-%, bezogen auf die Gesamt-Zusammensetzung des Mittels oder der Zubereitung, betragen, insbesondere aber 0,1 bis 1 Gew.-%, besonders bevorzugt aber 0,25 bis 0,75 Gew.-%. Bei Veiwendung von Insektenwachshydrolysaten und/oder Insektenwachsfraktionen, insbesondere solchen, die einen hohen Gehalt an (ω-1)-Hydroxysäuren mit 10 bis 20 Kohlenstoffatomen oder deren Salzen aufweisen und die beispielsweise nach dem vorstehenden Isolierungsverfahren erhältlich sind, entspricht dies etwa einem Gehalt von 0,5 bis 65 Gew.-%, insbesondere 2 bis 35 Gew.-% und besonders bevorzugt 2,5 bis 20 Gew.-% an der jeweiligen Insektenwachsfraktion.

Als zweckmäßig hat es sich erwiesen, den pH-Wert der Zubereitungen und der kosmetischen, dermatologischen oder medizinischen Formulierung im Bereich von 2 bis 12 einzustellen, vorzugsweise von 4,5 bis 9, insbesondere aber im Bereich von pH 5 bis pH 7.

Gegenstand der Erfindung sich auch die kosmetischen, dermatologischen oder medizinischen Zubereitungen, insbesondere kosmetische, dermatologische oder medizinische Mittel auf Basis üblicher kosmetischer, dermatologischer oder medizinischer Trägerstoffe sowie ein oder mehrerer Wirkstoffe, wobei der Wirkstoff eine erfindungsgemäße, ionenbindende oder ionenkomplexierende (ω-1)-Hydroxysäure bzw. eine die erfindungsgemäße (ω-1)-Hydroxysäure enthaltende Insektenwachsfraktion ist.

Geeignet sind beispielsweise die bekannten kosmetischen, dermatologischen oder medizinischen Zubereitungen wie O/W-Emulsionen, W/O-Emulsionen, Cremes, Lotionen, Milchen, Salben, Fettsalben, Gele, Tinkturen, Solubilisate, Lösungen, Suspensionen, ölige Zubereitungen oder feste, beispielsweise stiftförmige Zubereitungen, Puder oder Sprays in allen Formen.

Die Kosmetika können in bekannter Weise Trägerstoffe, Hilfsstoffe und Zusatzstoffe enthalten. Es sind z.B. wasserbindende Stoffe, Verdicker, Füllstoffe, Parfüm, Farbstoffe, Tenside, Lichtschutzmittel, Öle, Fette, Wachse, Sonnenschutzmittel, UV-A-Blocker, UV-B-Blocker, Vitamine, Proteine, Insektenrepellentien, Stabilisatoren, Antioxidantien, Konservierungsmittel, Alkohole, keratolytisch oder proteolytisch wirksame Substanzen, Wasser und/oder Salze, beispielsweise Puffersalze oder Puffergemische zur pH-Einstellung.

Bevorzugte erfindungsgemäße Kosmetika sind solche, die ein Lichtschutzmittel enthalten und Sonnenschutzpräparate, z.B. mit UV-A- und UV-B-Filtern. Solche Kosmetika neigen zu Verfärbungen, die sich erfindungsgemäß verhindern lassen.

Unerwartet zeigen die erfindungsgemäßen Wirkstoffe ein so hohes Ionenbindungsvermögen und Ionenkomplexierungsvermögen, daß sie EDTA in Kosmetika zu ersetzen vermögen. Außerdem wirkt der sich bildende Komplex in Kosmetika überraschenderweise nicht nachteilig in diesen Mitteln.

Die erfindungsgemäßen Wirkstoffe bewirken, daß keine Verfärbungen in Kosmetika auftreten, die durch Schwermetallionen hervorgerufen werden. Weiterhin besteht eine synergistische Wirkung zwischen antimikrobiell wirkenden Substanzen und den erfindungsgemäßen Wirkstoffen. Auch findet keine Oxydation von Farb- und Riechstoffen, die durch Schwermetallionen katalysiert wird, statt.

Ein besonderer Vorteil der erfindungsgemäßen Wirkstoffe im Vergleich zu den Eigenschaften von z.B. EDTA zeigt sich in der guten Verträglichkeit und der guten biologischen Abbaubarkeit z.B. in Kläranlagen, Vorflutern und Gewässern, so daß es nicht zu einer Anreicherung dieser Stoffe in der Natur kommen kann.

Die erfindungsgemäßen ionenbindenden oder ionenkomplexierenden Wirkstoffe zeichnen sich durch eine gute Hautverträglichkeit und eine gute Wirkung aus. Auch die hergestellten Präparate besitzen gute Wirkung und Verträglichkeit. Eine sehr gute Hautverträglichkeit zeigt sich insbesondere dann, wenn der Wirkstoff eine eine erfindungsgemäße (ω-1)-Hydroxysäure enthaltende Insektenwachsfraktion ist, die gleichzeitig einen Gehalt an unverzweigten Fettsäuren mit einer Kettenlänge von C₁₈ bis C₂₆ oder deren Salzen aufweist, wobei die (ω-1)-Hydroxysäuren und/oder die genannten Fettsäuren sowohl einzeln als auch im Gemisch vorliegen können.

Zur Parfümierung sind insbesondere solche Substanzen und Parfümöle geeignet, die stabil sind und die Haut nicht reizen.

Die Erfindung wird in den Beispielen erläutert. Die Erfindung ist aber nicht auf die genannten Zubereitungen und Mittel, Hilfsstoffe und Trägersubstanzen beschränkt.

Die erfindungsgemäßen Wirkstoffe oder deren Salze können als solche oder in gelöster Form oder als Suspension (z.B. als wäßrige, alkoholische oder alkoholisch-wäßrige Lösung oder Suspension) den übrigen Bestandteilen der Formulierungen zugesetzt werden.

Zubereitungen, die, in denen erfindungsgemäße ionenbindende und komplexierende Wirkstoffe enthalten sind, können topische Zubereitungen sein, beispielsweise kosmetische und dermatologische topische Zubereitungen oder aber auch übliche Arzneimittel-Darreichungsformen.

Die ionenbindenden und komplexierenden Wirkstoffe gemäß der Erfindung können vorzugsweise in Zubereitungen eingesetzt werden, die in Form von Granulaten, Kapseln, Pillen, Tabletten, Filmtabletten, Dragees, Sirupen, Emulsionen, Suspensionen, Dispersionen, Aerosolen und Lösungen sowie Flüssigkeiten oder auch als Zäpfen oder Vaginalkugeln vorliegen. Die Arzneimittel-Darreichungsformen können dabei wie üblich zusammengesetzt sein.

Die jeweils einzusetzenden Mengen an kosmetischen, dermatologischen oder medizinischen Trägerstoffen und Parfüm können in Abhängigkeit von der Art des jeweiligen Produktes vom Fachmann durch einfaches Ausprobieren leicht ermittelt werden.

Ansonsten sind die üblichen Maßregeln für das Zusammenstellen von kosmetischen, dermatologischen oder medizinischen Formulierungen zu beachten, die dem Fachmann geläufig sind.

Alle Prozentangaben oder Teile beziehen sich, soweit nicht anders angegeben, auf das Gewicht, insbesondere auf das Gesamtgewicht der Zubereitungen.

Die folgenden Beispiele dienen dazu, die Erfindungen zu beschreiben, ohne daß beabsichtigt ist, die Erfindungen auf diese Beispiele zu beschränken.

### Beispiel 1

### W/O-Crème

| | |
|---|---|
| Paraffinöl | 10,00 |
| Ozokerit | 4,00 |
| Vaseline | 4,00 |
| pflanzliches Öl | 10,00 |
| Wollwachsalkohol | 2,00 |
| Aluminiumstearat | 0,40 |
| Chinawachssäuren | 5,00 |
| Parfüm, Konservierungsstoffe | q.s. |
| Wasser, VES | ad 100,00 |

### Beispiel 2

### W/O-Lotion

| | |
|---|---|
| Paraffinöl | 25,00 |
| Siliconöl | 2,00 |
| Ceresin | 1,50 |
| Wollwachsalkohol | 0,50 |
| Bienenwachssäuren | 2,50 |
| Parfum, Konservierungsstoffe | q.s. |
| Wasser, VES | ad 100,00 |

### Beispiel 3

### O/W-Lotion

| | |
|---|---|
| Paraffinöl | 5,00 |
| Isopropylpalmitat | 5,00 |
| Cetylalkohol | 2,00 |
| Bienenwachs | 2,00 |
| Ceteareth-20 | 2,00 |
| PEG-20-Glycerylstearat | 1,50 |
| Glycerin | 3,00 |
| Bienenwachssäuren | 10,00 |
| Parfum, Konservierungsstoffe | q.s. |
| Wasser, VES | ad 100,00 |

### Beispiel 4

### O/W-Crème

| | |
|---|---|
| Pflanzliches Öl | 10,00 |
| Cetylalkohol | 2,00 |
| Glycerinmonostearat | 1,50 |
| PEG-30-Glycerylstearat | 2,00 |
| Glycerin | 3,00 |
| Isopropylpalmitat | 5,00 |
| Carbopol 980 (neutralisiert) | 0,30 |
| Bienenwachssäuren | 10,00 |
| Parfum, Konservierungsstoffe | q.s. |
| Wasser, VES | ad 100,00 |

### Beispiel 5

### Salbe

| | |
|---|---|
| Vaseline | 36,00 |
| Ceresin | 10,00 |
| Zinkoxid | 4,00 |
| Pflanzliches Öl | 20,00 |
| Hummelwachssäuren | 5,00 |
| Parfum, Konservierungsstoffe | q.s. |
| Paraffinöl | ad 100,00 |

### Beispiel 6

### Hautöl

| | |
|---|---|
| Cetylpalmitat | 3,00 |
| C12-15- Alkylbenzoat | 2.00 |
| Polyisobuten | 10,00 |
| Squalan | 2,00 |
| Hummelwachssäuren | 1,00 |
| Parfum, Konservierungsstoffe | q.s. |
| Paraffinöl | ad 100,00 |

### Beispiel 7

### Badeöl

| | |
|---|---|
| Paraffinöl | 20,00 |
| PEG-40-hydriertes Rizinusöl | 5,00 |
| Chinawachssäuren | 2,50 |
| Parfum, Konservierungsstoffe | q.s. |
| Sojaöl | ad 100,00 |

### Beispiel 8

### Lippenstift

| | |
|---|---|
| Ceresin | 8,00 |
| Bienenwachs | 4,00 |
| Carnaubawachs | 2,00 |
| Vaseline | 40,00 |
| Hydriertes Rizinusöl | 4,00 |
| Bienenwachssäuren | 15,00 |
| Parfum, Konseivierungsstoffe | q.s. |
| Paraffinöl | ad 100,00 |

### Beispiel 9

### Pflegemaske

| | |
|---|---|
| PEG-50 Lanolin | 0,50 |
| Glycerylstearat | 2,00 |
| Sonnenblumenkemöl | 3,00 |
| Bentonit | 8,00 |
| Kaolin | 35,00 |
| Zinkoxid | 5,00 |
| Chinawachssäuren | 2,50 |
| Parfum, Konservierungsstoffe | q.s. |
| Wasser | ad 100,00 |

### Beispiel 10

### Liposomenhaltiges Gel

| | |
|---|---|
| Lecithin | 6,00 |
| Pflanzliches Öl | 12,50 |
| Hydrolysiertes Kollagen | 2,00 |
| Xanthan Gum | 1,40 |
| Butylenglycol | 3,00 |
| Bienenwachssäuren | 1,50 |
| Parfum, Konservierungsstoffe | q.s. |
| Wasser | ad 100,00 |

### Beispiel 11

### Duschpräparat mit Rückfetter

| | |
|---|---|
| Cocoamidodiacetat | 10,00 |
| Natriumlaurylsulfat | 25,00 |
| Kalium Cocoyl Hydrolysiertes Kollagen | 5,00 |
| Macadamianußöl | 5,00 |
| Natriumchlorid | 0,60 |
| Bienenwachssäuren | 1,50 |
| Parfum, Konservierungsstoffe | q.s. |
| Wasser | ad 100,00 |

### Beispiel 12

### Seifenstück

| | |
|---|---|
| Na-Salz aus Talgfettsäuren | 60,00 |
| Na-Salz aus Kokosöl | 28,00 |
| Natriumchlorid | 0,50 |
| Hummelwachssäuren | 20,00 |
| Parfum, Konservierungsstoffe | q.s. |
| Wasser | ad 100,00 |

### Beispiel 13

### Syndetseife

| | |
|---|---|
| Natriumlaurylsulfat | 30,00 |
| Natriumsulfosuccinat | 10,00 |
| Kaliumcocoyl hydrolysiertes Kollagen | 2,00 |
| Dimethicon Copolyol | 2,00 |
| Paraffin | 2,00 |
| Maisstärke | 10,00 |
| Talcum | 10,00 |
| Glycerin | 3,00 |
| Bienenwachssäuren | 5,00 |
| Parfum, Konservierungsstoffe | q.s. |
| Wasser | ad 100,00 |

### Beispiel 14

### Haarpflegemittel

| | |
|---|---|
| TEA-Cocoyl hydrolysiertes Kollagen | 30,00 |
| Monoethanolaminlaurylsulfat | 25,00 |
| Mandelöl | 2,00 |
| Natriumchlorid | 1,00 |
| Chinawachssäuren | 2,50 |
| Parfum, Konservierungsstoffe | q.s. |
| Wasser | ad 100,00 |

### Beispiel 15

### Pflegeshampoo

| | |
|---|---|
| Natriumlaurylsulfat | 34,00 |
| Dinatriumlaurylsulfosuccinat | 6,00 |
| Cocoamidopropylbetain | 10,00 |
| Glycoldistearat | 5,00 |
| Hummelwachssäuren | 1,0 |
| Parfum, Konservierungsstoffe | q.s. |
| Wasser | ad 100,00 |

### Beispiel 16

### Haarkur

| | |
|---|---|
| Cetylalkohol | 5,00 |
| Caprylic/Capric Triglyceride | 3,00 |
| Petrolatum | 2,00 |
| Wollwachsalkohol | 0,50 |
| Chinawachssäuren | 2,50 |
| Parfum, Konservierungsstoffe | q.s. |
| Wasser | ad 100,00 |

### Beispiel 17

### Haarspülung

| | |
|---|---|
| Cocoamidopropylbetain | 5,00 |
| Cetylalkohol | 2,00 |
| Propylenglycol | 2,00 |
| Citronensäure | 0,30 |
| Bienenwachssäuren | 1,00 |
| Parfum, Konservierungsstoffe | q.s. |
| Wasser | ad 100,00 |

### Beispiel 18

### Haarfestiger

| | |
|---|---|
| Polyvinylpyrrolidon/Vinylacetat/Vinylpropionat-Copolymer | 5,00 |
| Ethanol | 45,00 |
| Bienenwachssäuren | 1,50 |
| Parfum, Konservierungsstoffe | q.s. |
| Wasser | ad 100,00 |

### Beispiel 19

### Frisiercrème

| | |
|---|---|
| Vaseline | 4,00 |
| Cetearylalkohol | 4,00 |
| PEG-40-hydriertes Rizinusöl | 2,00 |
| Isopropylpalmitat | 5,00 |
| Citronensäure | 1,00 |
| Bienenwachssäuren | 2,50 |
| Parfum, Konseivierungsstoffe | q.s. |
| Wasser | ad 100,00 |

### Beispiel 20

### Rasierschaum

| | |
|---|---|
| Stearinsäure | 7,00 |
| Natriumlaurylsulfat | 3,00 |
| Stearylalkohol | 1,00 |
| Glycerin | 5,00 |
| Triethanolamin | 3,60 |
| Chinawachssäuren | 2,50 |
| Parfum, Konservierungsstoffe | q.s. |
| Wasser | ad 100,00 |

### Beispiel 21

### Fußcrème

| | |
|---|---|
| Soluan 5 | 2,00 |
| Methylsalicylat | 5,00 |
| Caprylic/Capric Triglyceride | 10.00 |
| Stearinsäure | 5,00 |
| Cetylalkohol | 1,00 |
| Glycerin | 2,00 |
| Dimethicon | 1,00 |
| Carbopol 984 | 0,50 |
| Triethanolamin | 1,50 |
| Hummelwachssäuren | 5,00 |
| Parfum, Konservierungsstoffe | q.s. |
| Wasser | ad 100,00 |

### Beispiel 22

### Aerosolspray

| | |
|---|---|
| Octyldodecanol | 0,50 |
| Bienenwachssäuren | 0,50 |
| Parfum, Konservierungsstoffe | q.s. |
| Ethanol | ad 100,00 |

Die durch Zusammenmischung der jeweiligen Bestandteile erhaltene flüssige Phase wird zusammen mit einem Propan-Butan-Gemisch (2:7) im Verhältnis 39:61 in Aerosolbehälter abgefüllt.

### Beispiel 23

### Pumpspray

| | |
|---|---|
| PEG-40-Hydriertes Rizinusöl | 2,00 |
| Glycerin | 1,00 |
| Hummelwachssäuren | 1,00 |
| Parfum, Konservierungsstoffe | q.s. |
| Wasser | ad 100,00 |

### Beispiel 24

### Roll-on-Gel

| | |
|---|---|
| 1,3-Butylenglycol | 2,00 |
| PEG-40-Hydriertes Rizinusöl | 2,00 |
| Hydroxyethylcellulose | 0,50 |
| Chinawachssäuren | 2,50 |
| Parfum, Konservierungsstoffe | q.s. |
| Wasser | ad 100,00 |

### Beispiel 25

### Roll-on-Emulsion

| | |
|---|---|
| Tricetearethphosphat | 0,30 |
| Octyldodecanol | 2,00 |
| C12-15-Alkylbenzoat | 2,00 |
| C10-30-Alkylacrylat | 0,15 |
| Bienenwachssäuren | 1,00 |
| Parfum, Konservierungsstoffe | q.s. |
| Wasser | ad 100,00 |

### Beispiel 26

### Wachsstift

| | |
|---|---|
| Hydriertes Rizinusöl | 5,00 |
| Bienenwachs | 6,00 |
| Ceresin | 30,00 |
| C12-15-Alkylbenzoat | 17,00 |
| Hummelwachssäuren | 5,00 |
| Parfum, Konservierungsstoffe | q.s. |
| Octyldodecanol | ad 100,00 |

## Patentansprüche

1. Verfahren zur Trennung und Isolierung von Hydroxyfettsäuren und Fettsäuren aus Insektenwachshydrolysaten, welche nach Hydrolyseverfahren aus Insektenwachsen oder Insektenwachsgemischen erhalten werden, dadurch gekennzeichnet, daß man entweder
a) in einem ersten Schritt ein Insektenwachshydrolysat mit mindestens einem polaren Lösungsmittel behandelt, wobei sich ein Teil des Gemisches löst, die erhaltene Lösung von ungelösten Anteilen abtrennt, wobei die ungelösten Anteile aus vorwiegend nicht hydroxylierten, langkettigen höheren Fettsäuren bestehen und durch physikalische und/oder chemische Trennverfahren abge-trennt werden können, und daß man dann das Lösungsmittel aus der verbleibenden Lösung entfernt und einen weiteren Rückstand erhält, der die Hydroxyfettsäuren enthält, und gegebenenfalls in einem zweiten Schritt diesen erhaltenen Rückstand mit mindestens einem unpolaren Lösungsmittel behandelt, wobei sich eine Dispersion oder Suspension bildet, deren festen Anteil, der die Hydroxyfettsäuren enthält, davon abtrennt und die verbleibende flüssige Phase vom Lösungsmittel befreit, wodurch man einen Rückstand erhält, der vorwiegend aus nicht hydroxylierten, kurzkettigen höheren Fettsäuren besteht, oder in umgekehrter Reihenfolge
b) in einem ersten Schritt ein Insektenwachshydrolysat mit mindestens einem unpolaren Lösungsmittel behandelt, wobei sich eine Dispersion oder Suspension bildet, den festen Anteil, der die Hydroxyfettsäuren enthält, davon abtrennt und die verbleibende flüssige Phase vom Lösungsmittel befreit, wobei ein Rückstand entsteht der vorwiegend aus nicht hydroxylierten, langkettigen höheren Fettsäuren besteht, und gegebenenfalls in einem zweiten Schritt den verbliebenen festen Anteil mit den Hydroxyfettsäuren mit mindestens einem polaren Lösungsmittel behandelt, wobei sich ein Teil des festen Anteiles löst, die erhaltene Lösung von ungelösten Teilen, die vorwiegend aus nicht hydroxylierten höheren Fettsäuren kurzer Kettenlänge bestehen, befreit und von der Lösung das Lösungsmittel entfernt, wodurch man einen Rückstand erhält, der die Hydroxyfettsäuren enthält, und
c) gegebenenfalls zur Reinigung jeweils die zuvor erhaltenen Hydroxyfettsäuren oder Fettsäuren in wäßriger Lösung mit einer Base in eine Lösung ihrer Salze überführt, die Lösung von festen Anteilen befreit, dann nach Säurezugabe zur Lösung die Hydroxyfettsäure oder Fettsäure wiedergewinnt und isoliert, oder diese Reinigung gegebenenfalls auch am Anfang, vor den Schritten a) oder b), in entsprechender Weise mit dem Insektenwachshydrolysat vornimmt.

2. Verfahren gemäß Anspruch 1, dadurch gekennzeichnet, daß man in einem ersten Schritt ein Insektenwachshydrolysat mit mindestens einem polaren Lösungsmittel behandelt, wobei sich ein Teil des Gemisches löst, die erhaltene Lösung von ungelösten Anteilen abtrennt, wobei die ungelösten Anteile aus vorwiegend nicht hydroxylierten, langkettigen höheren Fettsäuren bestehen und daß man dann das Lösungsmittel aus der verbleibenden Lösung entfernt und einen weiteren Rückstand erhält, der die Hydroxyfettsäuren enthält, und gegebenenfalls in einem zweiten Schritt diesen erhaltenen Rückstand mit mindestens einem unpolaren Lösungsmittel behandelt, wobei sich eine Dispersion oder Suspension bildet, deren festen Anteil, der die Hydroxyfettsäuren enthält, davon abtrennt und die verbleibende flüssige Phase vom Lösungsmittel befreit, wodurch man einen Rückstand erhält, der vorwiegend aus nicht hydroxylierten, kurzkettigen höheren Fettsäuren besteht und gegebenenfalls zur Reinigung jeweils die zuvor erhaltenen Hydroxyfettsäuren oder Fettsäuren in wäßriger Lösung mit einer Base in eine Lösung ihrer Salze überführt, die Lösung von festen Anteilen befreit, dann nach Säurezugabe zur Lösung die Hydroxyfettsäure oder Fettsäure wiedergewinnt und isoliert, oder diese Reinigung gegebenenfalls auch am Anfang in entsprechender Weise mit dem Wollwachssäuregemisch vornimmt.

3. Verfahren gemäß Anspruch 1, dadurch gekennzeichnet, daß als polare Lösungsmittel Alkohole, insbesondere Methanol, Ethanol, n-Propanol, iso-Propanol, und/oder Ketone, insbesondere Aceton und/oder Ethylmethylketon, und/oder Gemische davon und als unpolare Lösungsmittel aliphatische und/oder cyclische Kohlenwasserstoffe, insbesondere Pentan, Hexan, Heptan und/oder Oktan und/oder die Isomeren dieser Lösungsmittel, Cyclohexan, und/oder alkylierte Aromaten, insbesondere Toluol und/oder Xylol, und/oder Gemische davon verwendet werden.

4. Verfahren gemäß Anspruch 1, dadurch gekennzeichnet, daß als Lösungsmittel ein oder mehrere überkritische Gase zur Extraktion Verwendung finden und diese nach Extraktion rückstandsfrei entfernt werden können.

5. (ω-1)-Hydroxyfettsäuregemische erhältlich nach dem Verfahren gemäß Anspruch 1.

6. (ω-1)-Hydroxyfettsäuregemische gemäß folgender Zusammensetzung (a) erhältlich nach dem Verfahren gemäß Anspruch 1:
| (a) | |
|---|---|
| **(ω-1)-Hydroxysäure** | **Gew.-%** |
| 1 (ω-1)-Hydroxy-hexadecansäure | 20 - 90 |
| 2 (ω-1)-Hydroxy-16-methylheptadecansäure | 1 - 15 |
| 3 (ω-1)-Hydroxy-tetradecansäure | 1 - 10 |
| 4 (ω-1)-Hydroxy-octadecansäure | 1 - 5 |
| 5 (ω-1)-Hydroxy-dodecansäure | 1 -2 |
| 6 weitere Homologe von 1 | 1- 20 |
| 7 Restbestandteile | ad 100 |

7. Kosmetische, dermatologische oder pharmazeutische Zubereitungen enthaltend eine oder mehrere (ω-1)-Hydroxyfettsäuren mit 10 bis 20 Kohlenstoffatomen und/oder deren Salze.

8. Verwendung von Gemischen nach Anspruch 5 oder 6 zur Bekämpfung von Mikroorganismen und deren kosmetischen oder pathologischen Auswirkungen, wobei die (ω-1)-Hydroxyfettsäuren sowohl einzeln als auch im Gemisch vorliegen können.

9. Kosmetische, dermatologische oder pharmazeutische Zubereitungen mit einem Gehalt an desodorierend wirkenden Gemischen nach Anspruch 5 oder 6, wobei die (ω-1)-Hydroxyfettsäuren sowohl einzeln als auch im Gemisch vorliegen können.

10. Fettsäuregemische erhältlich nach dem Verfahren gemäß Anspruch 1.

11. Kosmetische, dermatologische oder pharmazeutische Zubereitungen enthaltend eine oder mehrere unverzweigte Fettsäuren mit einer Kettenlänge von C₁₈ bis C₂₆ erhältlich nach dem Verfahren gemäß Anspruch 1 und/oder deren Salze.

12. Verwendung von Gemischen nach Anspruch 10 als hautpflegende und/oder barriererestaurierende Wirkstoffe in kosmetischen, dermatologischen oder medizinischen Zubereitungen, wobei die unverzweigten Fettsäuren sowohl einzeln als auch im Gemisch vorliegen können.
